# EUROPEAN PATENT APPLICATION

(11) **EP 4 592 678 A1**
(43) Date of publication of application: **30.07.2025**
(21) Application number: 24154199.4
(22) Date of filing: 26.01.2024
(51) Int. Cl.: G01N 33/543, B01L 3/00, G01N 27/74

(54) **METHOD AND DEVICE FOR DETECTING A CONCENTRATION OF AT LEAST ONE ANALYTE IN A SAMPLE**

(71) Applicant: Technische Universität München, 80333 München (DE)
(72) Inventor: HAYDEN, Oliver, 85368 Moosburg (DE); LEUTHNER, Moritz, 81539 München (DE); BRÄNDLE, Franziska, 87700 Memmingen (DE)
(74) Representative: Lucke, Andreas

(57) **Abstract**

The invention relates to a method for detecting a concentration of an analyte (10) in a sample (20), the method comprising: mixing capture elements (30), preferably capture elements comprising beads, more preferably comprising magnetic beads, into the sample (20), the capture elements(30) having capture binder elements (35,36,37); binding the analyte (10) to at least one capture element (30) via a capture binder element (35, 36, 37) such that at least one analyte bonded capture element (30) is formed; flowing the sample (20) through a channel (40) having a substrate (42), the substrate (42) having surface binder elements (44, 45, 46); moving the capture elements (30) to the substrate (42) with an external force (6o), preferably with a magnetic force; rolling the capture elements (30) along/on the substrate (42) and changing the velocity of said at least one analyte bonded capture element (30) by a binding interaction between a surface binder element (44, 45, 46) of the substrate (42) and the analyte (10) bound by the respective capture binder element (35, 36, 37); determining a concentration of the analyte (10) using a sensor unit (70) by determining a change of velocity of the capture elements (30) along the channel and/or by determining a depletion of the capture elements (30) along the channel (40), and/or determining binding kinetics, preferably binding constants, between the analyte (10) and the capture binder elements (35, 36, 37) and/or between the analyte (10) and the surface binder elements (44, 45, 46), wherein the binding kinetics are determined using the sensor unit (70) by determining the change of velocity of the capture elements (30) along the channel (40). The invention further relates to a device (100).

## Description

### FIELD OF THE INVENTION

The invention relates to a method for detecting a concentration of at least one analyte in a sample, and to a device for detecting a concentration of at least one analyte in a sample.

### BACKGROUND OF THE INVENTION

In clinical chemistry, the detection and quantification of extracellular vesicles (EVs) as well as other vesicular analytes are challenging tasks. Manual workload, missing standardization, ultracentrifugation and large capital invest for central laboratory equipment are required to perform these assays. However, these assays cannot be performed with unprocessed patient samples, e.g., whole blood. They usually employ expensive signal readout devices based on optical labeling, such as for fluorescence flow cytometry, and, due to the dependence on workflow breaking centrifugation, cannot be integrated into point-of-care applicable workflows. Additionally, the typical dynamic concentration range of reported assays is in the order of 2-3 concentration log scales, which is insufficient to cover multiple vesicular biomarkers for multiplexed analysis at the same time.

Such assays may be classified as heterogeneous, homogeneous or competitive assays.

Heterogeneous assays are a type of biochemical analysis that involves the separation of unbound components from the bound analyte of interest. In these assays, the target molecule, such as a protein or nucleic acid, is immobilized on a solid support, such as microbeads or a microplate well, while the unbound components are washed away. The detection of the bound analyte is achieved by using a labeled probe, such as an antibody or a fluorescent molecule, which specifically binds to the immobilized target. This binding event can be quantified through various detection methods, such as fluorescence, luminescence, or absorbance measurements. These workflows always require a sequence with washing steps.

Homogeneous assays do not require a separation step to detect the target analyte. These assays are conducted in a homogeneous solution without the need for washing or removing unbound components. Instead, the detection signal is generated through specific interactions between the target analyte and two antibodies in close distance for a fluorescence resonance energy transfer (FRET) readout. However, sufficient optical transparency is required such that no distortion occurs of the FRET effect.

Competitive assays are a type of biochemical analysis that involve the competition between a labeled analyte and an unlabeled analyte for binding to a limited number of binding sites on a capture molecule. In these assays, the analyte of interest is typically labeled with a detectable marker, such as a fluorescent molecule or an enzyme. The labeled analyte competes with an unlabeled analyte in the sample for binding to the capture molecule. The amount of labeled analyte bound to the capture molecule is inversely proportional to the concentration of the unlabeled analyte present in the sample. By measuring the signal generated by the labeled analyte, the concentration of the unlabeled analyte can be quantified.

A method and device for detecting a concentration of an analyte is disclosed in WO 2006/134546 A2. The method of WO 2006/134546 A2 is a competitive assay. There, magnetic capture beads may have binder elements to bind to analytes in a sample. Further, the analytes bound by the capture beads may further bind to surface binder elements of a substrate.

Known immunoassays for vesicular analytes require a labelling step and need multiple antibodies per analyte for binding at different sites to discriminate from unspecific binding and background effects. Further, such immunoassays are usually not qualified for Point-of-Care-Testing (POCT). Additionally, known immunoassays cannot analyze unprocessed patient samples with low optical transparency, require washing steps before analysis, do not cover multiple biomarkers (e.g., vesicles, protein, nucleic acid, cells) on a single read-out platform and employ expensive, fluorescence labelling and complex readout systems to discriminate from complex matrix effects. Moreover, known immunoassays do not allow for multiplexing without sensor arrays or serialized workflow solutions, thereby driving consumable costs.

### SUMMARY OF THE INVENTION

The drawbacks of the known assays are overcome by a method according to claim 1 and by a device according to claim 11. The dependent claims relate to particularly favorable embodiments of the invention.

A first aspect of the invention relates to a method for detecting a concentration of at least one analyte in a sample, the method comprising:
- mixing capture elements, preferably capture elements comprising beads, more preferably comprising magnetic beads, into the sample, the capture elements having capture binder elements;
- binding the analyte to at least one capture element via a capture binder element such that at least one analyte bonded capture element is formed;
- flowing the sample through a channel having a substrate, the substrate having surface binder elements;
- moving the capture elements to the substrate with an external force, preferably with a magnetic force;
- rolling the capture elements along/on the substrate and changing the velocity of said at least one analyte bonded capture element by an interaction, preferably by a binding interaction, between a surface binder element of the substrate and the analyte bound by the respective capture binder element;
- determining a concentration of the analyte using a sensor unit by determining a change of velocity of the capture elements along the channel and/or by determining a depletion of the capture elements along the channel, preferably by differential counting of the capture elements, and/or
determining binding kinetics, preferably binding constants, between the analyte and the capture binder elements and/or between the analyte and the surface binder elements, wherein the binding kinetics are determined using the sensor unit by determining the change of velocity of the capture elements along the channel.

By moving the capture elements to the substrate and rolling the capture elements along/on the substrate, the method allows to differentiate capture elements bound to the analyte from capture elements not bound to the analyte. Thus, the analyte concentration in the sample may be determined. The method further allows for non-optical determination of the concentration, so that a preprocessing may not be necessary. The concentration of analyte in opaque samples may be determined. Moreover, the concentration may be determined over a large range. Further, the present invention allows s for multiplexing, and/or heterogeneous assays, such that analytes of different type and/or kind and/or class in the sample may be analyzed, e.g., proteins, vesicles/vesicular material, cells/cellular material, chemicals, DNA, RNA and/or antibodies (serology).

Note that, in contrast to this, in the above mentioned WO 2006/134546 A2, the sample is not flown through a channel. Further, WO 2006/134546 A2 does not disclose an external force, e.g., a magnetic force, for moving capture elements to the substrate, nor discloses rolling of the capture elements along/on the substrate, nor determining the concentration of the analyte by determining a change and/or a decrease of velocity of the capture elements along the channel, and/or by determining a depletion of the capture elements along the channel. Rather, WO 2006/134546 A2 determines the concentration by relating a binding rate of the analyte to the substrate to changes in a magnetic field which are registered when the magnetic (capture) bead with the analyte binds to surface binder elements.

In the invention, the sample may be or may comprise a liquid sample. The sample may be or may comprise blood and/or urine. The analyte may be or may comprise a vesicle, a vesicle structure, one or more exosomes, mitochondria, and the like. In some embodiments, the sample may comprise or may contain more than one kind and/or type of analyte, at least one of which may be of interest for detecting its concentration. In other words, one or more of the kinds and/or types of analytes may be analytes for which the concentration in the sample is to be determined and/or detected. The analyte may comprise a ligand, e.g., an antigen. Different types and/or kinds of analyte may have different ligands and/or antigens.

A capture element binder element and/or a surface binder element may be or may comprise one or more antibodies, respectively. A capture binder element may be or may comprise the same antibody as a surface binder element. An antibody may bind to the analyte, and/or to a ligand, e.g., an antigen, of the analyte. Different types and/or kinds of capture binder elements may comprise e.g., different antibodies, and/or may be configured to bind to different kinds and/or types of analyte. Different types and/or kinds of surface binder elements may comprise, e.g., different antibodies, and/or may be configured to bind to different kinds and/or types of analyte. In some embodiments, it may be provided that the capture binder elements of at least one, multiple or all capture elements are of the same type and/or kind as at least some, multiple or all of the surface binder elements. Alternatively or additionally, the capture binder elements of at least one, multiple or all capture elements may match at least some, multiple or all of the surface binder elements, such that the respective capture binder elements and the surface binder elements form matched antibody pairs. In some embodiments, a capture binder element is or comprises a tetraspanin, e.g., CD81, CD63 and/or CD9. In some embodiments, a surface binder element is or comprises a tetraspanin, e.g., CD81, CD63 and/or CD9. In some embodiments, a capture binder element is or comprises an anti-Her2neu antibody and/or an anti-IL6 antibody. In some embodiments, an analyte is or comprises Her2neu+ EV, IL6, and/or biotin. In some embodiments, a substrate binder element is or comprises an anti-Her2neu antibody, an anti-IL6 antibody, and/or avidin.

An interaction between a surface binder element and the analyte bound by a surface binder element may comprise a binding interaction between the respective surface binder element and the analyte. The capture binder elements and/or the surface binder elements may be chosen such that they may bind to a specific, chosen and/or desired analyte. Particularly, if the sample comprises more than one type and/or kind of analyte, the capture binder elements and/or the surface binder elements may be chosen such as to bind to one of the analytes for which the concentration may be determined and/or tested, and/or which shall be assayed.

Mixing the capture elements into the sample may comprise or may be equal to introducing the capture elements into the sample. In some embodiments, multiple or all capture elements bond to analyte in the sample, e.g. depending on the concentration of analyte in the sample, the number of capture elements, the kind and/or type of analyte, and/or the kind and/or type of the capture binder elements of the respective capture elements.

The binding kinetics, e.g. binding constants, between the analyte and binder elements, e.g. the capture binder elements or the surface binder elements, can be determined by determining the change of velocity of the capture elements along the channel. The larger the change of velocity and/or the depletion, the stronger the binding interaction and/or the binding rate. Thus, the method allows for measuring and/or determining binding interactions, e.g. binding constants, of different analytes. In some embodiments, the method may be used to analyze, determine or tabulate the binding kinetics of analytes, e.g. analytes for which the binding kinetics are not known or only tentatively known. Further, the method may be used for checking, validating or verifying known binding kinetics.

The capture elements may be or may comprise beads. The beads may be or comprise magnetic beads. The capture elements and/or the beads be magnetic. The capture elements and/or beads may be at least partially made of or comprise a magnetic material. In some embodiments, moving the magnetic capture elements and/or the magnetic beads to the substrate comprises pulling or attracting the magnetic capture elements and/or the magnetic beads to the substrate. Alternatively or additionally, moving the magnetic capture elements and/or the magnetic beads to the substrate may comprise pushing the magnetic capture elements and/or the magnetic beads to the substrate. The external force may be a magnetic force provided by a magnetic field. In some embodiments, the magnetic field is a static magnetic field. Alternatively, the magnetic field may be varied, e.g., over time and/or along the channel. In some embodiments, the external force comprises an acoustic force and/or gravity. In these cases, the capture elements do not necessarily need to be magnetic. The external force may be configured to move the capture elements towards the substrate. Additionally, capture element movement from the external force may dominate over Brownian motion and movement by diffusion. Thus, the capture elements may be reliably moved in a direction specified by the external force, e.g., reliably pulled or attracted to (a wall of) the channel. Because of the external force, the movement and/or attraction of the capture elements towards (a wall of) the channel may be substantially directed, instead of, e.g., a random walk due to diffusion. Thereby, the path traveled by the capture elements when moving towards (a wall of) the channel may be shorter, and/or at least shorter on average, than compared to the prior art as, e.g., of WO 2006/134546 A2. A contact between the capture elements and the substrate, and/or a contact force, may be substantially due to the external force. In some embodiments, the external force is generated by an external force generator.

In some embodiments, multiple or all capture elements are moved, pushed and/or pulled to the channel and/or the substrate. It may be preferred that all capture elements are moved, pushed and/or pulled to the channel and/or the substrate. The external force may be configured suitable for moving all capture elements to the channel and/or the substrate. When one or more capture elements is/are moved to the channel and/or the substrate, the capture element(s) may contact the channel and/or the substrate.

It may be provided that the capture elements are detected by the sensor unit. In some embodiments, the capture elements comprise or consist of a metal or an alloy. Alternatively or additionally, the capture elements may comprise or may consist of glass or plastic. It may be provided that the capture elements are coated or plated. For instance, the capture elements may have a core comprising a first material, e.g., a suitable plastic, glass or metal, and may be coated or plated with another material, e.g., a suitable plastic, glass or metal. The plastic may be or may comprise a polymer. Alternatively or additionally, the capture elements may comprise or consist of a biological material. For instance, the capture elements may have an inner material comprising a first material, e.g., suitable polymers, glass, oil, protein, vesicular material, cellular material or metal, and may have an outer material comprising a second material, e.g., suitable polymers, glass, oil, protein, vesicular material, cellular material or metal.

The capture elements may be or may comprise beads. For instance, the beads may be metallic, and/or at least partially or fully made of a metal. It may be provided that the capture elements are spherical, but their form is not necessarily limited thereto.

In some embodiments, the capture elements comprise or consist of cellular and/or vesicular material. Additionally or alternatively, the capture elements may be or comprise stabilized droplets and/or cells. In some embodiments, the capture binder elements are or correspond to an outer surface of the capture element, e.g. to a wall of a droplet and/or a cell. Alternatively or additionally, the capture binder elements may be or may correspond to the outer material of the capture elements.

The flow through the channel, e.g., the flow with which the capture elements and/or the sample is flown through the channel, may be a laminar flow. However, the flow is not necessarily limited thereto. Depending on, e.g., the dimensions of the channel, the flow velocity, the viscosity of the flow and/or of the sample, the smoothness of the surface of the channel, and/or other relevant parameters, the flow may be at least partially turbulent or may undergo a turbulent transition.

In some embodiments, multiple or all capture elements roll along/on the substrate. Rolling on or rolling along the substrate does not necessarily mean that a respective capture element rolls on/along the full length of the substrate. Rolling on or rolling along the substrate may mean that a respective capture element rolls on/along some length of the substrate, which may be very short or minimal compared to a length of the substrate in some embodiments. Additionally or alternatively, rolling on or rolling along the substrate may be facilitated with, but not necessarily only with, the external force and/or the laminar flow. In some embodiments, the rolling is facilitated, supported, aided and/or caused by the external force. It may be provided that the movement and/or the speed of the capture element, in particular the rolling on the substrate, depend(s) on the external force, e.g., on its amplitude, amount and/or power. Alternatively or additionally, the rolling may be facilitated, supported, aided and/or caused by the flow through the channel. It may be provided that the movement and/or the speed of the capture element, in particular the rolling on the substrate, depend(s) on the flow, e.g., on its flow velocity, e.g., its mean flow velocity and/or flow rate. In some embodiments, the length along which the capture element rolls or may roll on/along the substrate, and/or its traveling path on/along the substrate, depend(s) on the external force and/or on the flow, and/or is specified or determined by the external force and/or by the flow. The movement of a capture element on or along the substrate may depend on the external force and/or on the flow, or may be specified or determined by the external force and/or by the flow.

When rolling on or rolling along the substrate, a capture element may at least partially contact the substrate. When rolling on or rolling along the substrate, the capture element may rotate while at least partially contacting the substrate. When rolling on or rolling along the substrate, the capture element may be rotating while moving on/along the substrate. A rolling movement may consist of or may comprise a translational movement and a rotational movement. A rolling movement may be or may correspond to a superposition of a translational movement and a rotational movement. When a capture element is rolling on or along the substrate, a sliding of the capture element may be negligible.

It may be provided that the velocity of at least one, multiple or all of the analyte bonded capture elements is changed and/or reduced when rolling on or along the substrate. Changing the velocity of the capture elements may comprise decreasing a velocity of at least one, of multiple or of all capture elements. It may be provided that the velocity of capture elements, which are not bound to analyte, is not changed and/or reduced, or at least not substantially changed and/or reduced, when rolling on or along the substrate. Changing and/or reducing the velocity may include that a capture element may be stuck or immobilized on the substrate. Changing and/or reducing the velocity of at least one of the analyte bonded capture elements may comprise immobilizing said at least one of the analyte bonded capture elements by bonding the surface binder element to the analyte bound by the capture binder element.

The sensor unit may comprise at least one sensing element. In a preferred embodiment, determining the concentration of the analyte comprises determining a time-of-roll between a first sensing element and a second sensing element. In some embodiments, the first sensing element is arranged upstream of the substrate. The second sensing element may be arranged downstream of the substrate and/or downstream of the first sensing element. It may be provided that the first sensing element is or comprises a magnetic field sensor, and/or a magnetic sensor half bridge. The second sensing element may be or may comprise a magnetic field sensor, and/or a magnetic sensor half bridge.

The time-of-roll may be defined as the time required for a capture element, or a group of capture elements, to roll along/on the substrate between two defined points or regions on and/or upstream and/or downstream of the substrate, e.g., between two sensing elements (e.g. between the first sensing element and the second sensing element), and/or between entering and leaving a region on the substrate.

The sensor unit and/or the sensing elements may be or may comprise a device configured for impedance sensing and/or magnetoresistive sensing. The sensor unit and/or the sensing elements may detect and/or may register a capture element passing over, along and/or through the sensor unit and/or sensing elements. It may be provided that the sensor unit and/or the sensing elements measure/measures and/or determine/determines the size of a capture element. Alternatively or additionally, the sensor unit and/or the sensing elements may be or may comprise a coulter counter.

In a preferred embodiment, the external force is a magnetic force generated by a static magnetic field, and the sensor unit may comprise at least one sensor configured to detect and/or to determine a magnetic field. When a capture element, particularly a magnetic capture element and/or a magnetic bead, passes the sensor unit, it may be provided that the magnetic field as detected and/or determined by the sensor unit changes, such that the (e.g., magnetic) capture element may be detected and/or its velocity may be determined.

In a preferred embodiment, the sample is flown or driven by a syringe, a syringe pump, and/or a metering pump. The syringe, the syringe pump and/or the metering pump may be fluidically connected to the channel, e.g., at or to an inlet of the channel. The syringe, the syringe pump and/or the metering pump may exert a force onto the sample such that the velocity of the sample is substantially constant when flowing into or through the channel. Thus, a velocity of the capture elements upstream and/or downstream of the substrate, and/or at the inlet of the channel, may be known. The velocity of the capture elements may be determined, detected and/or measured by the sensor unit downstream of the substrate. Then, the change and/or decrease of velocity of the capture elements may be determined by comparing the velocity as imposed by the syringe, the syringe pump and/or the metering pump, e.g., at the inlet, and the velocity as determined by the sensor unit.

In a preferred embodiment, determining a depletion of the capture elements comprise or is carried out by differential counting of the capture elements. Differential counting may be carried out by determining a difference of the number of capture elements counted and/or registered by two sensing elements. Additionally or alternatively, differential counting may be carried out by determining a difference of the number of capture elements counted and/or registered by one sensing element and a known capture element count before the sample with the capture elements reaches the substrate. A depletion of capture elements may occur when one or more analyte bonded capture elements are stuck or immobilized on the substrate. In some embodiments, a single sensing element is provided and/or is sufficient for carrying out the differential counting, as the number of capture elements mixed into the sample may be known. Then, the difference between capture elements mixed into the sample and capture elements registered by the sensing element may be determined, such that the number of capture elements stuck or immobilized on the substrate upstream of the sensing element may be determined.

Thus, in some embodiments a non-optical determination of the analyte concentration is possible. Hence, the method and the device according to the invention allow for assays where the sample does not need to be processed before carrying out the method. Further, the sample may be opaque.

In a preferred embodiment, determining the concentration of the analyte comprises an optical detection. The optical detection may comprise capturing a movement and/or a position of the capture elements spatially and/or temporally resolved in a field of view. By determining the position and/or the movement of capture elements on the substrate, the time-of-roll and/or the differential counting may be determined optically. The optical detection may be carried out by one or more optical devices, e.g. by one or more cameras. Each optical device and/or camera may have a field of view. In some embodiments, more than one optical device and/or more than one camera is provided, such as to have a larger total field of view at sufficient resolution. The fields of view of at least two optical devices and/or cameras may at least partially overlap. In some embodiments, the fields of view of at least two optical devices and/or cameras don't overlap. It may be provided that a first optical device and/or camera is arranged such that its field of view may cover a region upstream of the substrate, and a second optical device and/or camera is arranged such that its field of view covers a region downstream of the substrate. The optical detection may detect one or more of color, size, luminescence and granularity of the capture elements. Thereby, in some embodiments, multiplexing is enabled.

In a preferred embodiment, the external force, e.g. a magnetic force provided by a magnetic field, and a flow velocity of the sample are adjusted such that at least one of the capture elements rolls and/or is rolled along/on the substrate. For instance, in some embodiments, the capture elements, when moved towards the substrate, contact the channel and/or the substrate at substantially similar positions, or at least in a defined region on the channel and/or the substrate. Thereby, e.g., the length and/or the distance of the rolling on the substrate may be fixed, or at least may be specified more accurately. In some embodiments, the flow velocity is chosen such that the analyte bonded capture elements are only slowed down when rolling along/on the substrate, but not stuck or immobilized.

In a preferred embodiment, the external force, e.g., a magnetic force provided by a magnetic field, is adjusted such that the capture elements are pushed away from the substrate. In some embodiments, the external force is adjusted such that the capture elements are pushed away from the substrate, preferably after determining the concentration of the analyte. Thereby, the channel and/or the substrate may be prepared for another analysis or assay, and/or the capture element bound analyte used for further analysis with different methods. For instance, capture elements stuck on the substrate may be released from the substrate, which capture elements may then be used, e.g., for PCR (polymerase chain reaction). Alternatively or additionally, the temperature can be increased to minimize unspecific binding on the substrate or to achieve dissociation of the capture elements. In particular, when the binding is a DNA-DNA, RNA-DNA or DNA-PNA binding, the strength of the binding may be varied and/or decreased by increasing the temperature. In some embodiments, the temperature may be increased such that it approaches a corresponding melting temperature of the binding or is larger than a corresponding melting temperature of the binding, e.g., a corresponding melting temperature of a respective DNA-DNA binding, RNA-DNA binding and/or DNA-PNA-binding.

In a preferred embodiment, in the channel a laminated flow is flown between the sample and the substrate such as to separate the sample from the substrate. The capture elements may be moved by the external force, e.g., by a magnetic force generated by a magnetic field, from the sample through the laminated flow to the substrate. Viewed in a longitudinal section of the channel, a height of the laminated flow may be larger than the largest dimension, e.g., the diameter, of the capture elements. In some embodiments, more than one laminated flow is flown over the substrate, wherein the laminated flows may be substantially layered above each other, e.g., in a direction perpendicular to the surface of the substrate. The laminated flow may prevent debris to obstruct or to hinder the rolling of the capture elements along/on the substrate. The laminated flow, e.g., may minimize interference from hematocrit. In some embodiments, the laminated flow may be or may comprise a sheath flow.

In a preferred embodiment, at least one, multiple or all of the capture elements has/have at least two different capture binder elements each. The different capture binder elements may be configured to bind to different analytes. Thus, the method allows for multiplexing.

In a preferred embodiment, at least two of the capture elements have different sizes and different capture binder elements, wherein the different capture binder elements may be configured to bind to different analytes. Alternatively or additionally, at least two of the capture elements may have different magnetic moments and different capture binder elements, wherein the different capture binder elements may be configured to bind to different analytes. Alternatively or additionally, at least two of the capture elements may have different optical properties and different capture binder elements, wherein the different capture binder elements may be configured to bind to different analytes. In some embodiments, it may be provided that the capture elements comprise at least two groups of capture elements and the capture elements of each group may have the same capture binder elements. At least two capture elements, and/or the at least two groups of capture elements may differ in at least one or multiple of size, color, luminescence, granularity and magnetic moment. Thus, the method allows for multiplexing.

In a preferred embodiment, the substrate has at least two different surface binder elements, wherein the different surface binder elements may be configured to bind to different analytes. In some embodiments, it may be provided that the different surface binder elements are grouped into homogeneous groups, preferably separated by sensing elements. Thus, the method allows for multiplexing.

In some embodiments, the capture binder elements of at least one or all capture elements are of the same type and/or kind as at least one or all surface binder elements.

The method allows for multiplexing, and/or heterogeneous assays. With the method, analytes of different type and/or kind and/or class in the sample may be analyzed, e.g., proteins, vesicles/vesicular material, cells/cellular material, chemicals, DNA, RNA, antibodies (serology), and/or the concentration of multiple analytes in the sample may be determined. Different capture binder elements may differ in type and/or kind, e.g., may comprise or may be different antibodies. Different surface binder elements may differ in type and/or kind, e.g., may comprise or may be different antibodies. In some embodiments, different capture binder elements differ in number of capture binder elements arranged on respective capture element surfaces.

It may be provided that an orientation and/or a rolling direction of the capture elements are/is varied when the capture elements are rolling on or along the substrate. Thus, a larger fraction of the surface of the capture elements is coming into contact with the surface binder elements when rolling along/on the substrate, facilitating the bonding of surface binder elements to analytes bound to capture binder elements of the rolling capture elements. Thereby, the accuracy of the method may be improved, since capture elements bound to analytes may be better differentiated from those not bound to analyte.

A second aspect of the invention relates to a device for detecting a concentration of at least one analyte in a sample, the device comprising a channel, wherein the channel has a substrate, the substrate having surface binder elements. The device is configured such that, when the sample that comprises the analyte and capture elements with capture binder elements flows through the channel, an external force moves the capture elements to the substrate. At least one of the capture elements is bound to the analyte via a capture binder element forming an analyte bonded capture element. The capture elements are moved by the external force such that, when the capture elements flow through the channel, the capture elements roll along/on the substrate. The surface binder elements are configured to bind to the analyte and/or to reduce the velocity of said at least one analyte bonded capture element by an interaction, preferably by a binding interaction, between at least one of the surface binder elements and the analyte bound by the respective capture binder element when said at least one analyte bonded capture element rolls along/on the substrate. The device comprises a sensor unit configured to determine a change of velocity of the capture elements along the channel, and/or to determine a depletion of the capture elements along the channel, preferably by differential counting of the capture elements, and/or to determine binding kinetics, preferably binding constants, between the analyte and the capture binder elements and/or between the analyte and the surface binder elements.

It may be provided that when the capture elements are flown through the channel, multiple or all of the capture elements roll along/on the substrate.

The device may be configured to carry out a method according to the first aspect of the invention. The device may have one, multiple or all advantages of the inventive method as described herein.

In some embodiments, the device may comprise the sample. In some other embodiments, the device may not comprise the sample. In some embodiments, the device may comprise the capture elements. In some other embodiments, the device may not comprise the capture elements.

In some embodiments, the device comprises an external force generating device which is configured to generate the external force. The external force generating device may be or may comprise a magnetic field generating device configured to generate a magnetic field, such that the external force may be a magnetic force. The external force, e.g., the magnetic force, may be configured to move the capture elements to the substrate. The capture elements may be or may comprise magnetic beads, and/or may be magnetic.

The sensor unit may comprise at least one sensing element. In a preferred embodiment, the sensor unit comprises a first sensing element and a second sensing element, wherein the sensor unit may be configured to determine the concentration of the analyte by determining a time-of-roll between the first sensing element and the second sensing element. In some embodiments, the first sensing element is arranged upstream of the substrate. The second sensing element may be arranged downstream of the first sensing element and/or the substrate. The first sensing element may be or may comprise a magnetic field sensor, and/or a magnetic sensor half bridge. The second sensing element may be or may comprise a magnetic field sensor, and/or a magnetic sensor half bridge.

In a preferred embodiment, the external force generating device is a magnetic field generating device, e.g., a permanent magnet or the like. The external force may be a magnetic force. The magnetic field generating device may generate a static magnetic field. The sensor unit may comprise at least one sensor configured to detect and/or to determine a magnetic field and/or a magnetic field change. When a capture element, particularly a magnetic capture element and/or magnetic bead, passes the sensor unit, it may be provided that the magnetic field as detected and/or determined by the sensor unit changes, such that the (e.g., magnetic) capture element may be detected and/or its velocity may be determined.

In a preferred embodiment, the sensor unit is or comprises an optical device. The optical device may be configured to determine the concentration of the analyte by capturing a movement and/or a position of the capture elements spatially and/or temporally resolved in a field of view. The optical device may comprise one or more cameras. Each optical device and/or camera may have a field of view. In some embodiments, more than one optical device and/or more than one camera is provided, such as to have a larger total field of view at sufficient resolution. The fields of view of at least two optical devices and/or cameras may at least partially overlap. In some embodiments, the fields of view of at least two optical devices and/or cameras don't overlap. It may be provided that a first optical device and/or camera is arranged upstream of the substrate and/or such that its field of view may cover a region upstream of the substrate, and a second optical device and/or camera is arranged downstream of the substrate and/or such that its field of view may cover a region downstream of the substrate. The optical device may be configured to detect one or more of color, size, luminescence and granularity of the capture elements. Thereby, in some embodiments, multiplexing is enabled.

In a preferred embodiment, the substrate has at least two different surface binder elements, wherein the different surface binder elements may be configured to bind to different analytes. It may be provided that the different surface binder elements are grouped into homogeneous groups separated by sensing elements.

In a preferred embodiment, the channel and/or the substrate have/has a mechanical and/or magnetic pattern. The mechanical and/or magnetic pattern may be or may comprise a mechanical and/or magnetic chevron pattern. The mechanical and/or magnetic pattern may be or may comprise a mechanical and/or a magnetic structure. The mechanical and/or magnetic pattern may be configured to vary an orientation and/or a rolling direction of the capture elements when the capture elements are rolling along/on the substrate. Thus, a likelihood or chance of analyte bound to capture binder elements for contacting and/or interacting with surface binder elements is increased.

In a preferred embodiment, the channel may be tapered. When the channel is tapered, its cross-section may vary along its length. When the cross-section increases, the flow velocity may decrease. The channel may be tapered such that its cross-section increases in flow direction. When the channel is tapered, a driving force imparted by the flow on the capture elements may decrease, and the velocity of an analyte bonded capture element may be further decreased. In some embodiments, the likelihood of an analyte bonded capture element to be immobilized or stuck may be increased. In some embodiments, the capture elements are guided and/or directed to the sensing unit by the tapering of the channel.

In a preferred embodiment, the device comprises a second external force generating device which is configured to generate a second external force. The second external force generating device is/may be arranged downstream of the sensor unit. In some embodiments, the second external force generating device is or comprises a second magnetic field generating device and the second external force is a magnetic force. The second magnetic field generating device may be or may comprise a permanent magnet. The second external force generating device may be configured to generate the second external force varying in amplitude and/or spatial direction. The second external force may vary over time. It may be provided that the second external force generating device is a sorting unit for sorting capture elements. For instance, the second external force may be used to sort capture elements, e.g., sorting the capture elements downstream of the sensor unit. By sorting, the capture elements may be sorted into different groups. Capture elements of different groups may be used for further analysis or diagnostics.

### SHORT DESCRIPTION OF THE FIGURES

The invention is further disclosed and detailed in view of the following figures:
- Fig. 1:: a schematic view of a device according to an embodiment of the invention;
- Fig. 2:: another schematic view of a device according to an embodiment of the invention;
- Fig. 3:: a schematic view of capture elements with capture binder elements;
- Fig. 4:: a flow diagram showing a method according to an embodiment of the invention;
- Fig. 5:: a schematic view of a device according to an embodiment of the invention, at a first time t1;
- Fig. 6:: the embodiment of Fig. 5, at a second time t2;
- Fig. 7:: an exemplary output of the sensor unit of the embodiment of Figs. 5 and 6;
- Fig. 8:: a comparison of the embodiment of Figs. 5 to 7 to conventional assays;
- Fig. 9:: a schematic view of a device according to another embodiment of the invention;
- Fig. 10:: a comparison of the embodiment of Fig. 9 to conventional assays;
- Fig. 11:: a schematic view of a device according to yet another embodiment of the invention;
- Fig. 12:: an exemplary output of the sensor unit of the embodiment of Fig. 11;
- Fig. 13:: a comparison of the embodiment of Figs. 11 and 12 to conventional assays;
- Fig, 14:: a schematic view of a device according to an embodiment of the invention with sheath flow;
- Fig. 15:: a schematic view of a device according to an embodiment of the invention with multiplexing;
- Fig. 16:: a schematic view of a device according to another embodiment of the invention with multiplexing;
- Fig. 17:: a schematic view of a device according to yet another embodiment of the invention with multiplexing;
- Fig. 18:: a schematic view of a device according to an embodiment of the invention, with a pattern for varying a direction and/or an orientation of the capture elements;
- Fig. 19:: a schematic view of a device according to another embodiment of the invention, with a pattern for varying a direction and/or an orientation of the capture elements;
- Fig. 20:: a schematic view of a device according to yet another embodiment of the invention, with a pattern for varying a direction and/or an orientation of the capture elements;
- Fig. 21:: a schematic view of a device according to an embodiment of the invention, with a tapered channel; and
- Fig. 22a:: exemplary concentrations of analyte, as determined by methods according to the invention as function of change of velocity of the capture elements;
- Fig. 22b:: exemplary concentrations of analyte, as determined by methods according to the invention as function of the ratio the ratio of immobilized and/or stuck capture elements;
- Fig. 23a:: a comparison of exemplary concentrations of analyte, as determined by different methods according to the invention as function of the ratio of immobilized and/or stuck capture elements; and
- Fig. 23b:: a comparison of exemplary concentrations of analyte, as determined by different methods according to the invention as function of the ratio of immobilized and/or stuck capture elements.

### DESCRIPTION OF PREFERRED EMBODIMENTS

It is to be understood that both the foregoing general description and the following description are exemplary and explanatory only and are not restrictive of the methods and devices described herein. In this application, the use of the singular may include the plural unless specifically state otherwise. Also, the use of "or" means "and/or" where applicable or unless stated otherwise. Those of ordinary skill in the art will realize that the following description is illustrative only and is not intended to be in any way limiting. Other embodiments will readily suggest themselves to such skilled persons having the benefit of this disclosure. Reference will now be made in detail to various implementations of the example embodiments as illustrated in the accompanying drawings. Same reference numerals may denote same objects in the figures.

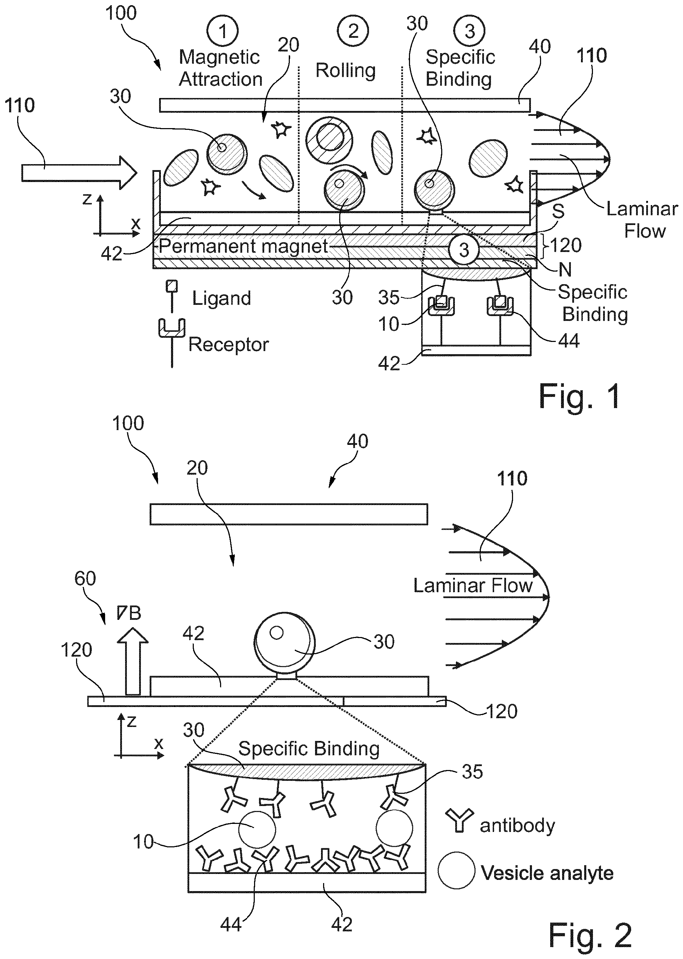

Figs. 1 and 2 show exemplary embodiments of a device 100 according to the invention. The device 100 may be considered a test device for carrying out an assay. The device 100 may be used to determine a concentration of an analyte 10 in a sample 20. The device 100 may be configured to carry out a method according to the invention.

The sample 20 may comprise a liquid or may be a liquid sample 20. For example, the sample 20 may be or may comprise blood or urine. The sample 20 may contain one or more types and/or kinds of analyte 10 to be analyzed. The analyte 10 may be or may comprise Interleukin 6 (IL-6). The analyte 10 may be or may comprise a vesicle. The analyte 10 may be or may comprise a vesical structure, one or more exosomes, mitochondria, and the like. However, other and/or different analytes, and/or types and/or kinds of analyte 10, may be analyzed and/or detected.

The device 100 comprises a channel 40. A sample 20 with analyte 10 and with at least one analyte bonded capture element 30, and/or with capture elements 30, may be flown through the channel 40. In particular, a flow 110 of the sample 20 with the capture elements 30 mixed therein may be flown through the channel 40. The flow 110 may be laminar. The capture elements 30 may be magnetic. The capture elements 30 may be or may comprise magnetic beads.

The channel 40 may be or may comprise a microfluidic channel. The channel 40 may have an inlet and an outlet for flowing the sample 20 through the channel 40. The channel 40 may have a cross-diameter in the z-y-plane (e.g., Figs. 1 and 2), through which cross-diameter the flow 110 may flow. The cross-diameter may be substantially, rectangular, oval, circular, or have any other suitable shape. For instance, a flow direction through the channel 40 may be in x-direction. In some embodiments, the channel 40 maybe arranged and/or maybe oriented such that its length-wise extension is substantially perpendicular to gravity. For instance, gravity may act in negative z-direction.

The channel 40 has a substrate 42. The substrate 42 may be arranged at an inner side wall of the channel 40, or may be part of an inner side wall of the channel 40. The substrate 42 may be arranged at a bottom of the channel 40. The substrate 42 may extend in a flow direction through the channel 40 (e.g., in x-direction in Figs. 1 and 2). The substrate 42 may be or may comprise a plane or a surface, and/or may extend in a direction perpendicular to its length-wise extension (e.g., in y-direction in Fig. 1 and 2). The substrate 42 may be arranged such that the flow 110 flows over/above the substrate 42. Gravity may act in negative z-direction (cf. e.g. Figs. 1 and 2), and the substrate 42 may be arranged such that it supports the flow 110. However, different arrangements and/or orientations of the channel 40, and/or the substrate 42, are possible.

The substrate 42 comprises surface binder elements 44. The surface binder elements 44 are arranged on a surface of the substrate 42 along which the sample 20 flows and/or along/on which the capture elements 30 may roll. The surface binder elements 44 may extend from the substrate 42. The surface binder elements 44 may bind to the analyte 10, and/or the analyte 10 may bind to the surface binder elements 44.

The surface binder elements 44 may be or may comprise antibodies for binding to the analyte 10. The analyte 10 may be or may comprise a ligand and/or an antigen to bind to the surface binder elements 44 and/or respective antibodies. The surface binder elements 44 may be chosen such that they may bind to a specific, chosen, and/or desired analyte 10. A capture binder element 35, 36, 37 may have the same antibody as a surface binder element 44, 45, 46. In some embodiments, a surface binder element 44, 45, 46 is or comprises a tetraspanin, e.g., CD81, CD63 and/or CD9. In some embodiments, a capture binder element 35, 36, 37 is or comprises an anti-Her2neu antibody and/or an anti-IL6 antibody. In some embodiments, an analyte 10 is or comprises Her2neu+ EV, IL6, and/or biotin. In some embodiments, a substrate binder element 42 is or comprises an anti-Her2neu antibody, an anti-IL6 antibody, and/or avidin.

The device 100 may further compromise an external force generating device 120. The external force generating device 120 may be or may comprise a magnetic field generating device 120. The external force generating device 120 may be configured to generate an external force 60. The external force 60 may be or may comprise a magnetic force, e.g., generated by a magnetic field, and/or an acoustic force. The magnetic field generating device may be configured to generate a magnetic field and/or a magnetic force. In some embodiments, the external force generating device 120 and/or the magnetic field generating device is arranged at the channel 40. The magnetic field generating device may be or may comprise a magnet, e.g., a permanent magnet. For instance, in the embodiment of Fig. 1, the magnetic field generating device comprises a magnetic north pole and a magnetic south pole. It may be provided that the magnetic field is a static magnetic field.

Alternatively or additionally, the magnetic field generating device may be or may comprise an electromagnet. However, the magnetic field generating device is not necessarily limited thereto. In some embodiments, e.g., when the magnetic field generating device is or comprises an electromagnet, it may be provided that the magnetic field is varied, e.g. locally or over time.

When the capture elements 30 are magnetic and/or comprise magnetic beads, the magnetic field and/or the magnetic field generating device may be configured to move the capture elements 30 to the substrate 42, e.g., by attracting the capture elements 30.

In some embodiments, the device 100 comprises more than one or multiple external force generating devices 120 and/or magnetic field generating devices, as shown in the embodiment of Fig. 2. In some embodiments, when the device 100 comprises more than one external force generating device 120 and/or more than one magnetic field generating device, the external force60, e.g., a magnetic force, generated by the respective external force generating devices 120 differs, e.g., in strength and/or orientation.

It may be provided that external force 60, e.g., the magnetic field and/or magnetic force, is varied or reversed. In some instances, the external force 60 is varied or reversed to release stuck and/or immobilized capture elements 30 from the substrate 42. In some embodiments, the channel 40 and/or the substrate 42 are/is freed from stuck and/or immobilized capture elements 30 when the analyte concentration has been determined and/or after completion of the analyte concentration analysis.

In some embodiments, e.g., in those of Figs. 1 and 2, the external force generating device 120, e.g., a magnetic field generating device 120, is arranged below the substrate 42, e.g., below the substrate 42 in z-direction. The external force generating device 120 may exert a pulling force onto the capture elements 30 flowing within the channel 40 and/or the capture elements 30 above the substrate 42. Thus, the capture elements 30 flowing in the channel 40 may be moved or may be moving to the substrate 42.

Additionally or alternatively, in some embodiments, one or more of the external force generating devices 120, e.g. one or more magnetic field generating device 120, is/are arranged above the substrate 42, e.g. at or above an upper sidewall of the channel 40 or may be arranged opposite the substrate 42. The external force generating device 120 may exert a pushing force onto the capture elements 30 flowing within the channel 40 and/or the capture elements 30 above the substrate 42. Thus, the capture elements 30 flowing in the channel 40 may be moved or may be moving to the substrate 42.

In some embodiments, the external force 60 is or comprises gravity. In some embodiments, it may be provided that the external force 60 is superposed by gravity, and/or includes gravity besides another force. If the external force 60 is or comprises gravity, in some embodiments the device 100 does not comprise an external force generating device 120. However, in some other embodiments the device 100 comprises an external force generating device 120 even in cases when the external force 60 is or comprises gravity. In some embodiments, the flow velocity and/or the capture elements 30 are chosen and/or configured such, e.g., the capture elements 30 may comprise a suitable material and/or may have a suitable size, that gravity is sufficient to move the capture elements 30 towards the substrate 42. In some other embodiments, gravity is not sufficient to cause or to provide said movement of the capture elements 30.

It may be provided that the external force 60, e.g., a magnetic field and/or a magnetic force, is adjusted, chosen or varied in combination with a flow velocity of the flow 110 such that the capture elements 30 may roll along/on the substrate 42. In particular, the flow velocity of the flow 110 and the external force 60 may be adjusted, chosen or varied such that all capture elements 30, or a chosen or specific fraction of the capture elements 30, may roll along/on the substrate 42. The flow velocity of the flow 110 and the magnetic field 60 may be adjusted, chosen or varied such that all capture elements 30, or a chosen or specific fraction of the capture elements 30, may contact the substrate 42 at or near a specified point along the flow direction (e.g., along the x-direction in the figures).

In some embodiments, the flow velocity of the flow 110 is chosen such that the analyte bonded capture elements 30 are only slowed down when rolling along/on the substrate 42, but not stuck or immobilized. In some embodiments, the flow velocity of the flow 110 is controlled or varied, e.g., depending on the size of the capture elements 30 and/or the strength of the binding interaction.

The external force 60 and/or the external force generating device 120 may be configured and/or arranged such that some, most or all of the capture elements 30 may contact an inner side wall of the channel 40 and may roll along/on said inner side wall, before rolling onto the substrate 42.

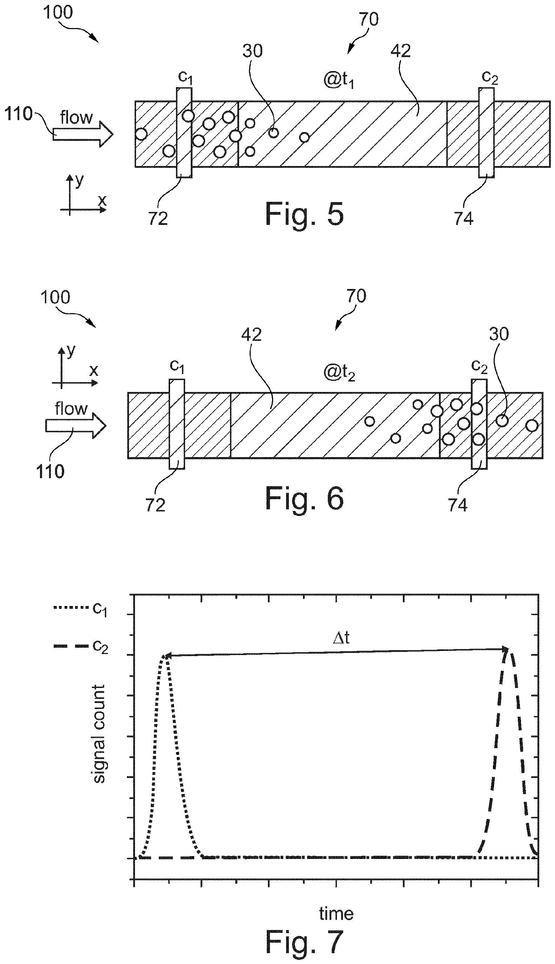

The device 100 comprises a sensor unit 70 (not shown in Figs. 1 and 2). Exemplary embodiments of the sensor unit 70 are described below with reference to Figs. 5 to 12. For instance, the sensor unit 70 of the embodiments of Figs. 1 and 2 (not shown in those figures) may correspond to or may be equal to one or more of the sensor units 70 as described hereafter and/or shown in Figs. 5, 7, 8, 11, 18, 19, 20, or 21.

The device 100 may comprise a control unit (not shown in the figures). The control unit may be or may comprise a computer unit. The control unit may be configured to control the external force generating device 120, the external force 60 and/or the flow velocity of the flow 110. The control unit may be configured to determine the concentration of the analyte 10 in the sample 20.

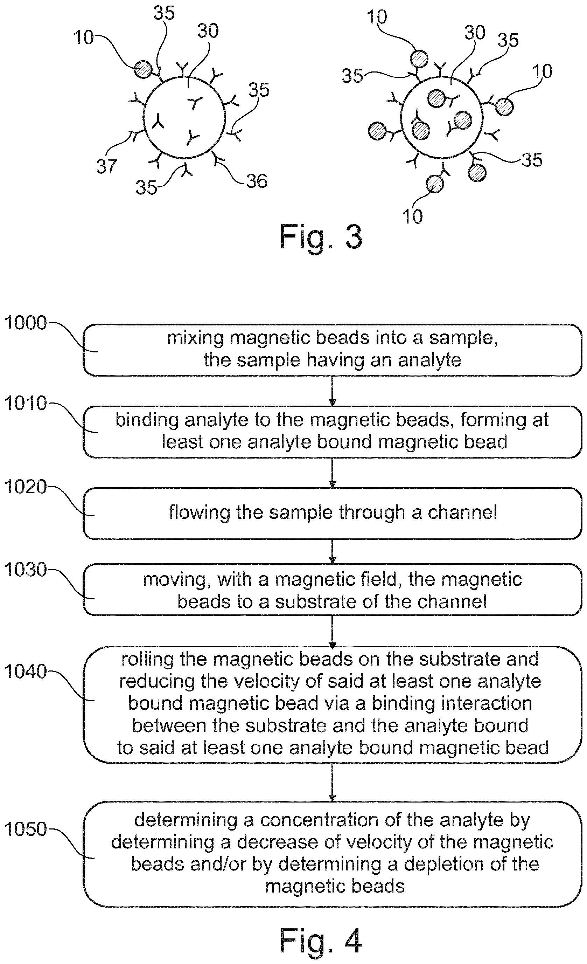

In Fig. 3, exemplary capture elements 30 are shown. The capture elements 30 may be spheres, or at least substantially spherical. However, the capture elements 30 are not necessarily limited thereto. Other shapes or forms may be employed. In some embodiments, the capture elements 30 may have diameters between 2 - 100 µm. In some embodiments, the capture elements 30 are magnetic. It may be provided that the capture elements are made of, consist of or comprise at least one or more of a metal, alloy, plastic and glass. It may be provided that a core of a capture element 30 is made of, consists of or comprises a first material, and that a surface of the capture element 30 is made of, consists of or comprises a second material. The first material may be different from the second material. In some embodiments, the capture elements 30 are coated and/or plated.

The capture elements 30 may have one or more capture binder elements 35, to which an analyte 10 may bind or may be bound. It may be provided that at least one capture element 30, or several, multiple or all capture elements 30, has different kinds and/or types of capture binder elements 35, 36, 37. For instance, a capture element 30 may have a first type of capture binder elements 35, a second type of capture binder elements 36, and/or a third type of capture binder elements 37. The number of types and/or kinds of capture binder elements 35, 36, 37 is not necessarily limited to three. In some embodiments, there may be more or less different types and/or kinds of capture binder elements 35, 36, 37. In some embodiments, it may be provided that at least one capture binder element, and/or multiple or all capture binder elements, has/have only one and/or a single type and/or kind of capture binder elements 35. In some embodiments, a capture binder element 35, 36, 37 is or comprises a tetraspanin, e.g., CD81, CD63 and/or CD9.

In some embodiments, the number of capture binder elements 35, 36, 37 per capture element 30 differs for different capture elements 30.

It may be provided that the number of capture binder elements 35, 36, 37 for each type and/or kind is larger than one, cf., e.g., Fig. 3. The capture binder elements 35, 36, 37 may extend from a surface of the capture element 30. The capture binder elements 35, 36, 37 may be arranged and/or may be distributed on the capture element 30 and/or its surface symmetrically, isotropically, and/or substantially over the full surface area of the capture element 30.

The capture binder elements 35, 36, 37 may be chosen with respect to the analyte 10 to be detected and/or to be analyzed. In some embodiments, it may be provided that when the capture element 30 has two or more types and/or kinds of capture binder elements 35, 36, 37, the method and/or the device 100 may be configured for multiplexing and/or heterogeneous assays, as described below.

The capture binder elements 35, 36, 37 may be or may comprise antibodies for binding to an analyte 10. When an analyte 10 is bound to at least one of the capture binder elements 35, 36, 37 of a capture element 30, an analyte bonded capture element 30 is formed. The analyte 10 may be or may comprise a ligand and/or an antigen to bind to a capture binder element 35, 36, 37 and/or a respective antibody. The capture binder element 35, 36, 37 may be chosen such that it may bind to a specific, chosen, to be determined or desired analyte 10. Particularly, when the sample 20 comprises more than one type and/or kind of analyte 10, the capture binder elements 35, 36, 37 may be chosen such as to bind to one of the analytes 10 for which the concentration may be determined or tested, and/or which shall be assayed. It may be provided that when the sample 20 comprises more than one type and/or kind of analyte 10, different kinds and/or types of capture binder elements 35, 36, 37 may be chosen such as to selectively bind to analytes 10 for which the concentration may be determined or tested, and/or which shall be assayed. In some embodiments, multiplexing for multiple analytes 10, e.g. multiple kinds and/or types of analyte 10, is provided, as described below.

In Fig. 4, steps of an exemplary embodiment of a method according to the invention are shown. The method according to the invention may comprise, may be, may correspond to or may be called a rolling bead sandwich assay.

In step 1000, capture elements 30 are mixed into a sample 20 comprising an analyte 10. The mixing may comprise introducing the capture elements 30 into the sample 20. The capture elements 30 may be or may comprise beads and/or magnetic beads.

In step 1010, at least one of the capture elements 30 binds to the analyte 10 in the sample 20. For binding to the analyte 10, the capture elements 30 have one or more capture binder elements 35, 36, 37. The capture binder elements 35, 36, 37 are configured to bind to the analyte 10. The analyte 10 may bind to the capture binder elements 35, 36, 37. The capture binder elements 35, 36, 37 may be or may comprise one or more antibodies, respectively. The antibodies may bind to the analyte 10, and/or a ligand, e.g., an antigen, of the analyte 10. The capture binder element 35, 36, 37 may be chosen such that it may bind to a specific, chosen, or desired analyte 10.

When the analyte 10 binds to a capture element 30, an analyte bonded capture element 30 may be formed, and/or a capture element 30 with the analyte 10 bound thereto may be designated as analyte bonded capture element 30. Not all capture elements 30 may have or must have analytes 10 bound to capture binder elements 35, 36, 37. There may be capture elements 30 in the sample 20, which are unbound to the analyte 10. However, in some embodiments, all capture elements 30 are analyte bonded capture elements 30 and/or have the analyte 10 bound to the capture binder elements 35, 36, 37.

In step 1020, the sample 20 is flown through a channel 40. The sample 20 flowing through the channel 40 contains or comprises the analyte 10, and at least one capture element 30 to which the analyte 10 is bound.

The channel 40 has a substrate 42. The substrate 42 comprises surface binder elements 44. The surface binder elements 44 are arranged on a surface of the substrate 42 along/on which the sample 20 flows and/or along/on which the capture elements 30 may roll. The surface binder elements 44 may bind to the analyte 10, and/or the analyte 10 may bind to the surface binder elements 44.

In step 1030, the capture elements 30 in the sample 20 flowing through the channel 40 are moved towards a substrate 42 of the channel 40 by means of an external force 60, e.g. by means of a magnetic field. The external force 60 may act on the capture elements 30, and may move the capture elements 30 towards or to a substrate 42 of the channel 40. In some embodiments, the magnetic field 60 exerts a (magnetic) force on the capture elements 30.

It may be provided that moving capture elements 30 towards or to the substrate 42 comprises, is or corresponds to pulling or attracting the capture elements 30 to the substrate 42. Alternatively or additionally, it may be provided that moving capture elements 30 towards or to the substrate 42 comprises, is or corresponds to pushing the capture elements 30 to the substrate 42.

In step 1040, when the capture elements 30 are moved to the substrate 42, the capture elements 30 roll along/on the substrate 42. It may be provided that the capture elements 30 are moved to the substrate 42 by the external force 60, e.g., by a magnetic force generated by a magnetic field, such as to contact the substrate 42 and/or to roll along/on the substrate 42. The capture elements 30 may be pushed on/onto the substrate 42 by the flow 110 of the sample 20, such that they may roll along/on the substrate 42. In some embodiments, all capture elements 30 entering the channel 40 are moved by the external force 60, e.g., pulled to the substrate 42, such that they roll along/on the substrate 42 when flowing through the channel 40. In some embodiments, a majority or at least a fraction of the capture elements 30 entering the channel 40 is moved by the external force 60, e.g., pulled to the substrate 42, such that they roll along/on the substrate 42 when flowing through the channel 40.

When a capture element 30, to which an analyte 10 is bound, rolls along/on the substate 42, the velocity of said capture element 30 may be changed. When the velocity is changed, the velocity may be reduced. The reduction of velocity may be due to interactions, e.g., binding interactions, between the analyte 10 that is bound to the capture element 30 and one or more surface binder elements 44. The binding interaction may be a so-called specific binding.

In some embodiments, changing and/or reducing the velocity comprises immobilizing at least one respective analyte bonded capture element 30. In some embodiments, at least one analyte bonded capture element 30 is slowed down, but not immobilized. It may be provided that the capture elements 30 without an analyte 10 bound thereto are not substantially slowed down when rolling along/on the substrate 42 (due to lack of any binding interaction described in the previous paragraph).

The number and/or the ratio of respective analyte bonded capture elements 30 immobilized and/or slowed down may depend, e.g., on the strength of the binding interaction between the surface binder elements 44 and the analyte 10 bonded to the capture element 30, and/or on the density and/or the position of surface binder elements 44 on the substrate 42 and/or on the concentration of the analyte 10 in the sample 20. For instance, more than one capture binder element 35, 36, 37 of a capture element 30 has an analyte 10 bound thereto, which - in some cases - may further reduce the rolling velocity of said capture element 30 along/on the substrate 42 as there may be multiple binding interactions between the respective bonded analytes 10 and the multiple surface binder elements 44.

In step 1050, a concentration of the analyte 10 in the sample 20 is determined, e.g., by determining a change and/or decrease of the velocity of the capture elements 30 flowing within the channel 40. The higher the analyte concentration, the higher the change and/or decrease of velocity, since the capture elements 30 rolling along/on the substrate may be slowed down the more analyte 10 is bound to the capture elements 30 due to an interaction, e.g., a binding interaction, between the surface binder elements 44 and the analyte 10 bound to the capture elements 30. Alternatively or additionally, the concentration of the analyte 10 in the sample 20 may be determined e.g. by determining a depletion of the capture elements 30 along the channel 40. A depletion of capture elements 30 may comprise an immobilization of at least some of the capture elements 30.

In an embodiment, the sample 20 is flown or driven by a syringe, a syringe pump, and/or a metering pump (not shown in the figures). The syringe, the syringe pump and/or the metering pump may be fluidically connected to the channel 40, e.g., at or to an inlet of the channel 40. The inlet may be arranged upstream of the substrate 42. The syringe, the syringe pump and/or the metering pump may exert a force onto the sample 20 such that the velocity of the sample 20 is substantially constant when flowing into or through the channel. Thus, a velocity of the capture elements 30 upstream of the substrate 42, and/or at the inlet of the channel 40, may be known. The velocity of the capture elements 30 may be determined, detected and/or measured by the sensor unit 70 downstream of the substrate 42. For instance, the velocity may be determined or measured by the sensor unit 70 as detailed below, e.g., by a sensing element 72, 74, 76 and/or by an optical device 78. Then, the change and/or decrease of velocity of the capture elements may be determined by comparing the velocity as imposed by the syringe, the syringe pump and/or the metering pump, e.g., at the inlet, and the velocity as determined by the sensor unit 70. In some embodiments, there is no sensing element 72, 74, 78 and/or optical device 78 upstream of the substrate 42, and/or a sensing element 72, 74, 78 and/or optical device 78 upstream of the substrate 42 is redundant.

Figs. 5 to 8 show an exemplary embodiment of a device 100 and a method according to the invention. The device 100 comprises a channel 40 with a substrate 42, e.g., a channel 40 and a substrate 42 as described above.

The device 100 may comprise an external force generating device 120, e.g., a magnetic field generating device (not shown in Figs. 5 and 6), which is configured to generate an external force 60, e.g. a magnetic force generated by a magnetic field (not shown in Figs. 5 and 6). Further, the device 100 comprises a sensor unit 70. The sensor unit 70 comprises two sensing elements 72, 74. The second sensing element 74 is arranged downstream of the first sensing element 72, e.g., arranged downstream in flow direction (for instance downstream in x-direction in the Figs. 5 and 6). The embodiment as shown in Figs. 5 to 8 may have at least one, several or all features and/or advantages of at least one other embodiment according to the invention.

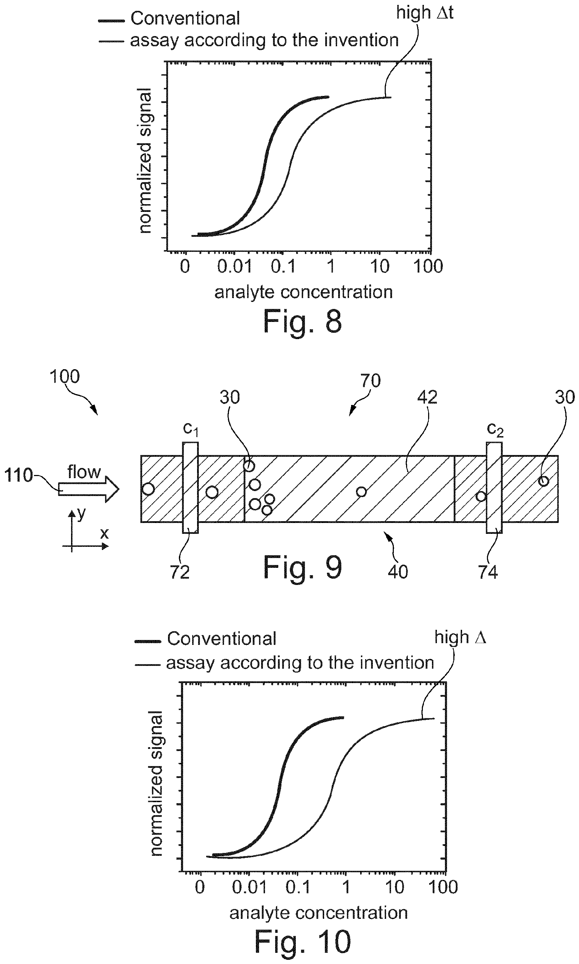

Fig. 5 shows a device 100 with the sample 20 comprising capture elements 30 flowing through the channel 40 at a first time t1. Fig. 6 shows the device 100 with a sample 20 comprising capture elements 30 flowing through the channel 40 at a second time t2. The second time t2 may be later than the first time t1. Fig. 7 shows an output of the sensor unit 70, and/or of the first sensing element 72 and the second sensing element 74, as a function of time. Fig. 8 shows a comparison of analyte concentrations detected and/or determined by a method according to the invention to analyte concentrations detected and/or determined by methods of the prior art.

In some embodiments, the sensor unit 70 and/or the sensing elements 72, 74 are/is or comprise/comprises a device configured for impedance sensing and/or magnetoresistive sensing. For instance, the capture elements 30 may be configured to be detected and/or sensed by impedance sensing and/or by magnetoresistive sensing. Alternatively or additionally, the sensor unit 70 and/or the sensing element 72, 74 may be or may comprise a coulter counter. In some embodiments, the first sensing element 72 and/or the second sensing element 74 are/is or comprise/comprises a magnetic field sensor, and/or a magnetic sensor half bridge, respectively. Thus, in some embodiments a non-optical determination of the analyte concentration is possible. Hence, the method and the device according to the invention allow for assays where the sample 20 does not need to be processed before carrying out the method. Further, the sample 20 may be opaque.

The sensor unit 70 and/or the sensing elements 72, 74 may detect and/or may register a capture element 30, e.g., a magnetic capture element 30, passing over, along or through the sensor unit 70 and/or the sensing elements 72, 74. It may be provided that the sensor unit 70 and/or the sensing elements 72, 74 measure/measure and/or determine/determines the size of a capture element 30.

The first sensing element 72 may be arranged upstream of the substrate 42 (cf., e.g., Figs. 5 and 6). In some embodiments, the first sensing element 72 and/or the second sensing element 74 are/is arranged over/above the substrate 42. The second sensing element 74 may be arranged downstream of the first sensing element 72 and/or downstream of the substrate 42 (cf., e.g., Figs. 5 and 6). The definition of "downstream" and "upstream" may be with respect to the flow direction of flow 110.

In some embodiments, e.g., in the embodiment of Figs. 5 to 8, the concentration of the analyte 10 and/or a decrease of the velocity of the capture elements 30 may be determined by determining a time-of-roll of the capture elements 30. The capture elements 30 may be magnetic.

The time-of-roll may be determined and/or may be measured between two sensing elements, e.g., between the first sensing element 72 and the second sensing element 74. However, it should be understood that, additionally or alternatively, a time-of-roll may be determined and/or may be measured between multiple sensing elements 72, 74, 76, and/or between two sensing elements 72, 74 separated by another sensing element 76. Multiple time-of-roll measurements and/or determinations may be carried out and alternatively or additionally at different temperatures. For instance, a first time-of-roll may be determined and/or may be measured between a first sensing element 72 and a second sensing element 74, and a second time-of-roll may be determined and/or may be measured between a third sensing element and a fourth sensing element. For instance, the first, second, third and fourth sensing element may be different sensing elements. However, it may be provided that, e.g., the third sensing element corresponds to or is equal to the first or the second sensing element 72, 74. Similarly, different arrangements of sensing elements 72, 74, 76, and/or time-of-roll measurements and/or determinations, are possible.

For instance, as depicted, e.g., in Fig. 5, a first sensing element 72 may detect and/or may register capture elements 30, e.g. magnetic capture elements 30, passing along and/or over the first sensing element 72, e.g. rolling along/on the substrate 42. A second sensing element 74 may detect and/or may register capture elements 30 passing along and/or over the second sensing element 72, e.g., rolling along/on the substrate 42, cf.,e.g., Fig. 6. When one or multiple capture elements 30 passes/pass along and/or over one of the sensing elements 72, 74, a corresponding signal may be outputted by the respective sensing element at a time of passing, cf., e.g., Fig. 7. The signal may be higher the more capture elements 30 pass at one instance of time, and/or the closer capture elements 30 are grouped or packed. Thus, at the first time t1, a first signal corresponding to at least one capture element 30 or a group of capture elements 30 passing the first sensing element 72 may be detected (cf. fig. 5 and fig. 7). Then, when the at least one capture element 30 or the group of capture elements 30 pass the second sensing element 74 downstream of the first sensing element 72, the second sensing element 74 may output a second signal at the second time t2 (cf., e.g., Fig. 6 and Fig. 7). The time difference Δt = t2- t1 may be used together with a known distance between the first sensing element 72 and the second sensing element 74 to determine a velocity of the at least one capture element 30 or the group of capture elements 30. The time-of-roll may be or may correspond to the time difference Δt.

The higher the analyte concentration in the sample 20, the higher the time difference Δt and the higher the velocity decrease. Thus, the analyte concentration may depend on and/or maybe correlated with and/or may be a function of the velocity decrease, and/or the velocity decrease may be employed to determine an analyte concentration in the sample 20.

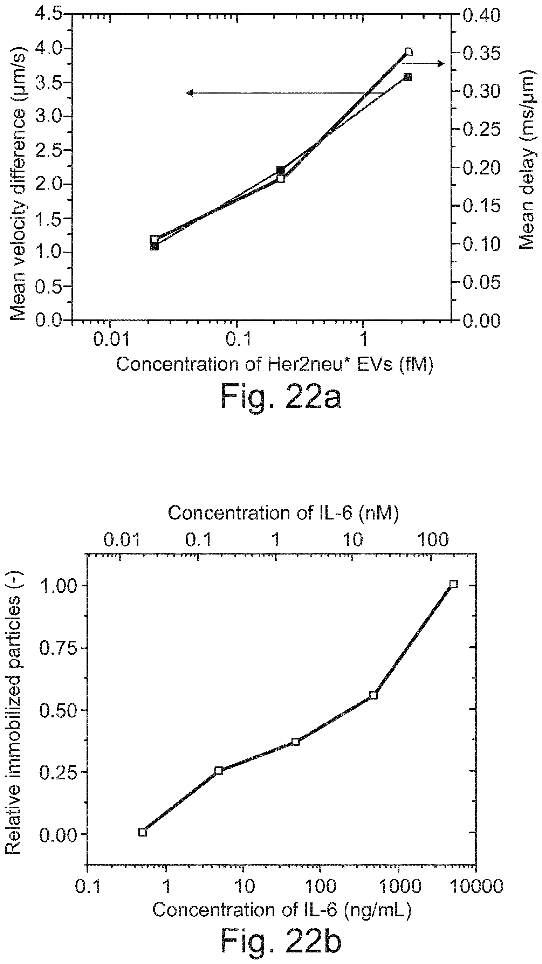

In Fig. 8, the method according to the invention is compared to conventional assays. Whereas conventional assays may detect and/or may determine analyte concentrations over 2 to 3 orders of magnitude, the method and the device according to the invention may detect and/or may determine analyte concentrations over 3 to 4 orders of magnitude, or even more. Thus, the method and the device according to the invention allow for a significantly improved detection and/or determination of the analyte concentration over a wide range, and provide assays with higher range of detection. Another example of concentrations as determined by a change of velocity of capture elements 30 is shown in Fig. 22a.

It may be provided that a reference velocity difference is given and/or determined, e.g., depending on a given flow velocity and/or for a given flow velocity. The reference velocity may be determined and/or given for a flow 110 without an analyte 10, i.e. for capture elements 30 unbound to an analyte 10 and/or not slowed down when rolling along/on the substrate 42. The velocity difference may be determined and/or given with respect to the reference velocity. In some embodiments, the reference velocity may be an average flow velocity of the flow 110.

Alternatively or additionally, optical methods may be used to determine the concentration of the analyte 10 and/or to determine the velocity of capture elements 30. The sensor unit 70 may be or may comprise one or more optical devices 78 (not shown in Figs. 5 and 6), e.g., one or more cameras. The sensor unit 70, the optical device 78 and/or the camera may take snapshots at specific times, and/or may capture photos or videos. The sensor unit 70, the optical device 78 and/or the camera may temporally and/or spatially capture the movement and/or the position of the capture elements 30. The resolution of the sensor unit 70, of the optical device 78 and/or of the camera may be temporally, and/or spatially, resolved. The sensor unit 70, the optical device 78 and/or the camera may take snapshots at specific times, and/or may capture photos or videos in a field of view. The field of view (not shown in Figs. 5 and 6) may encompass the substrate 42, fully or at least part of it. The field of view may be along the flow direction, e.g., in x-direction of Figs. 5 and 6.

Then, the velocity of the capture elements 30 may, e.g., be determined by comparing different positions of the capture elements 30 at different times and alternatively or additionally at different temperatures.

In some embodiments, more than one optical device 78 and/or more than one camera is provided, such as to have a larger total field of view 79 at sufficient resolution. It may be provided that different optical devices 78 and/or cameras have different fields of view 79, respectively. In some embodiments, at least two, multiple or all different fields of view 79 at least partially overlap. Alternatively or additionally, it may be provided that at least two, multiple or all fields of view 79 do not overlap. Different and/or overlapping fields of view 79 may form a composite field of view and/or a total field of view. It may be provided that a first optical device 78 and/or camera is arranged upstream of the substrate 42 and/or such that its field of view 79 may cover a region upstream of the substrate 42. A second optical device 78 and/or camera may be arranged downstream of the substrate 42 and/or such that its field of view 79 may cover a region downstream of the substrate 42.

The optical device 78 and/or the camera may detect one or more of color, size, luminescence and granularity of the capture elements 30. Thereby, in some embodiments, multiplexing is enabled.

Figs. 9 and 10 show another embodiment of a device 100 and a method according to the invention. Fig. 9 shows a device 100 with a sample 20 comprising capture elements 30 flowing through the channel 40. Figure 10 shows a comparison of analyte concentrations detected and/or determined by a method according to the invention to analyte concentrations detected and/or determined by methods of the prior art. The capture elements 30 may be magnetic, and/or may comprise magnetic capture elements, e.g., magnetic beads.

The device 100 comprises a channel 40 with a substrate 42, e.g., a channel 40 and a substrate 42 as described above. The device 100 may have an external force generating device 120, e.g. a magnetic field generating device (not shown in Fig. 9), which is configured to generate an external force 60, e.g. a magnetic force generated by a magnetic field (not shown in Fig. 9). Further, the device 100 comprises a sensor unit 70. The sensor unit 70 comprises two sensing elements 72, 74. The second sensing element 74 may be arranged downstream of the first sensing element 72, e.g., downstream in flow direction (for instance downstream in x-direction in Fig. 9). The embodiment as shown in Figs. 9 to 10 may have one, several or all features and/or advantages of at least one other embodiment according to the invention.

The concentration of the analyte 10 in the sample 20 may be determined by determining a depletion of the capture elements 30, e.g., of magnetic capture elements, along the channel 40, cf., e.g., the embodiment as shown in Figs. 9 and 10.

Determining the depletion may be carried out, e.g., by means of differential counting. For instance, it may be provided that the sensor unit 70, the first sensing element 72 and/or the second sensing element 74 may detect, may register and/or may count the capture elements 30, e.g., magnetic capture elements, passing through, along or over them. In some embodiments, the sensor unit 70 and/or the sensing elements 72, 74 are or comprise/comprises a device configured for impedance sensing and/or magnetoresistive sensing. The capture elements 30 may be configured to be detected and/or sensed by impedance sensing and/or by magnetoresistive sensing. Alternatively or additionally, the sensor unit 70 and/or the sensing element 72, 74 may be or may comprise a coulter counter. In some embodiments, the first sensing element 72 and/or the second sensing element 74 are or comprise/comprises a magnetic field sensor, and/or a magnetic sensor half bridge, respectively.

The differential counting may be determined and/or measured between two sensing elements, e.g., between the first sensing element 72 and the second sensing element 74, cf., e.g., Fig. 9. Differential counting may be based on comparing the count of capture elements 30 as counted by two different and/or distinct sensing elements 72, 74. It may be provided that a sensing element outputs a signal which may be used to count and/or correspond to countings of capture elements 30. "counting by a sensor unit and/or a sensing element" may comprise that the sensor unit and/or the sensing element outputs a suitable signal, and the counting is carried out by a control unit and/or by a computer unit. Alternatively or additionally, the differential counting may be repeated at different temperatures.

However, it should be understood that a differential counting may be determined and/or measured between multiple sensor elements 72, 74, 76, and/or between two sensor elements 72, 74 separated by another sensor element 76. Multiple differential countings may be carried out. For instance, a differential counting may be carried out by comparing the number of capture elements 30 counted by a first sensing element 72 and counted by a second sensing element 74, and a second differential counting may be carried out by comparing the counts of a third sensing element and of a fourth sensing element. For instance, the first, the second, the third and the fourth sensing element may be different sensing elements 72, 74, 76. However, it may be provided that, e.g., the third sensing element corresponds to or is equal to the first or the second sensing element 72, 74. Similarly, different arrangements of sensing elements 72, 74, 76 and/or differential countings are possible. A differential count may be or may correspond to a difference of the number of counted capture elements 30 of two respective sensing elements 72, 74.

A depletion of capture elements 30 may comprise an immobilization of at least some of the capture elements 30. For instance, when a capture element 30 with an analyte 10 bonded thereto is stuck and/or immobilized on the substrate 42, e.g. by one or more surface binder elements 44, the sensing elements 72, 74 may count a different number of capture elements 30. Since the number and/or the ratio of capture elements 30 that are immobilized and/or stuck on the substrate 42 may depend on the analyte concentration, the higher the differential count, the higher the analyte concentration. Thus, the analyte concentration may depend on, may be correlated with and/or may be a function of the differential counting, and/or the differential counting may be employed to determine an analyte concentration.

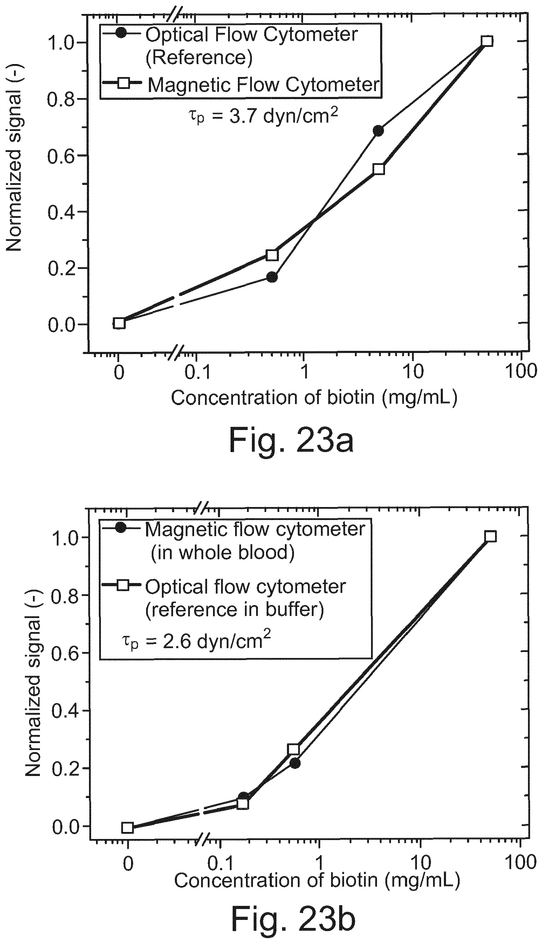

In Fig. 10 the method according to the invention is compared to conventional assays. Whereas conventional assays may detect and/or may determine analyte concentrations over 2 to 3 orders of magnitude, the method and the device according to the invention may detect and/or may determine analyte concentrations over 3 to 4 orders of magnitude, or even more. Thus, the method and the device according to the invention allow for a significantly improved detection and/or determination of the analyte concentration over a wide range, and provide assays with higher range of detection. It may be provided that the ratio and/or number of stuck and/or immobilized capture elements 30 of Fig. 22b, Fig. 23a and/or Fig. 23b is determined with differential counting.

Alternatively or additionally, optical methods may be used to determine the concentration of the analyte and/or to carry out the differential counting. The sensor unit 70 may be or may comprise one or more optical devices 78 (not shown in Fig. 9), e.g., one or more cameras. The sensor unit 70, the optical device 78 and/or the camera may take snapshots at specific times, and/or may capture photos or videos. The sensor unit 70, the optical device 78 and/or the camera may temporally and/or spatially capture the movement and/or the position of the capture elements 30. The sensor unit 70, the optical device 78 and/or the camera may take snapshots at specific times, and/or may capture photos or videos in a field of view 79.

In some embodiments, more than one optical device 78 and/or more than one camera is provided, such as to have a larger total field of view 79 at sufficient resolution. It may be provided that different optical devices 78 and/or cameras have different fields of view 79, respectively. In some embodiments, at least two, multiple or all different fields of view 79 may at least partially overlap. Alternatively or additionally, it may be provided that at least two, multiple or all fields of view 79 do not overlap. A field of view (not shown in Fig. 9) may encompass the substrate 42, fully or at least part of it. The field of view may be along the flow direction, e.g., in x-direction of Fig. 9. Different and/or overlapping fields of view 79 may form a composite field of view and/or a total field of view. It may be provided that a first optical device 78 and/or camera is arranged upstream of the substrate 42 and/or such that its field of view 79 may cover a region upstream of the substrate 42. A second optical device 78 and/or camera may be arranged downstream of the substrate 42 and/or such that its field of view 79 may cover a region downstream of the substrate 42. The optical device 78 and/or the camera may detect one or more of color, size, luminescence and granularity of the capture elements 30. Thereby, in some embodiments, multiplexing is enabled.

For instance, the movement and/or the position of the capture elements 30, e.g., magnetic capture elements, in the channel 40 and/or on the substrate 42, e.g., the rolling along/on the substrate 42, may be captured. Then, the velocity and/or the position of the capture elements 30 may, e.g., be determined by comparing different positions of the capture elements 30 at different times. Immobilized and/or stuck capture elements 30 may be identified and/or may be determined, e.g., by comparing the positions and/or the velocities at different times. The differential count may comprise, may correspond to and/or may be equal to the number of immobilized and/or stuck capture elements 30.

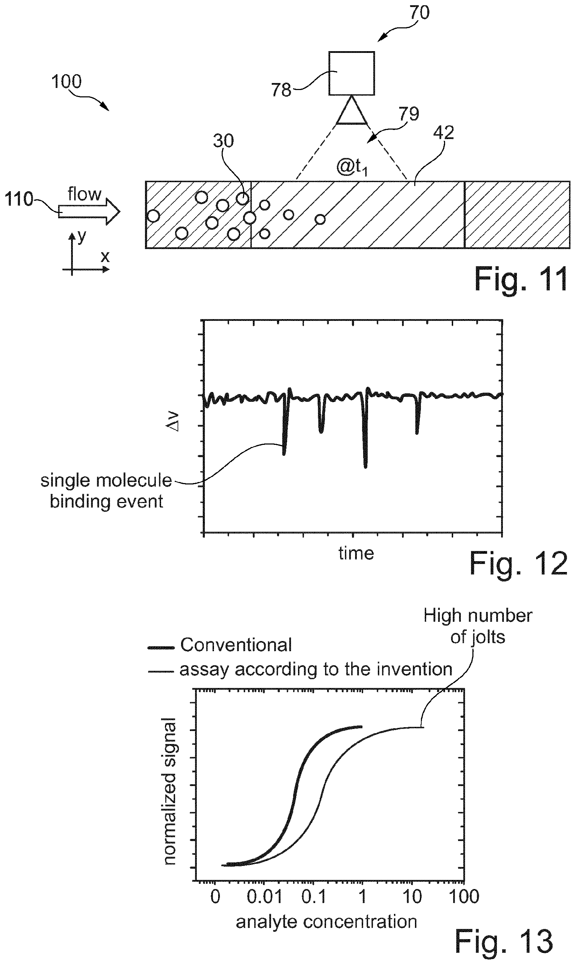

Figs. 11 to 13 show another embodiment of a device 100 and a method according to the invention. For instance, determining a concentration of the analyte 10 and/or a depletion of capture elements 30, e.g., of magnetic capture elements, may be carried out by detection of jolting of capture elements 30. Fig. 12 shows detected jolts, which occur when a capture element 30 is stuck and/or immobilized, e.g., on the substrate 42. Fig. 13 shows a comparison of analyte concentrations detected and/or determined by a method according to the invention to analyte concentrations detected and/or determined by methods of the prior art. The embodiment as shown in Figs. 11 to 13 may have one, several or all features and/or advantages of at least one other embodiment according to the invention.

Fig. 11 shows a device 100 with a sample 20 comprising capture elements 30 flowing through the channel 40. The device 100 comprises a channel 40 with a substrate 42, e.g., a channel 40 and/or a substrate 42 as described above. The device 100 may comprise an external force generating device 120, e.g., a magnetic field generating device (not shown in Fig. 9), which is configured to generate an external force 60 (not shown in Fig. 9), e.g., a magnetic force generated by a magnetic field. Further, the device 100 comprises a sensor unit 70. The sensor unit 70 comprises one or more optical devices 78. The optical device 78 may be or may comprise one or more cameras. The sensor unit 70, the optical device 78 and/or the camera may take snapshots at specific times, and/or may capture photos or videos. The sensor unit 70, the optical device 78 and/or the camera may temporally and/or spatially capture the movement and/or the position of the capture elements 30.

In some embodiments, more than one optical device 78 and/or more than one camera is provided, such as to have a larger total field of view 79 at sufficient resolution. It may be provided that different optical devices 78 and/or cameras have different fields of view 79, respectively. In some embodiments, at least two, multiple or all different fields of view 79 at least partially overlap. Alternatively or additionally, it may be provided that at least two, multiple or all fields of view 79 do not overlap. Different and/or overlapping fields of view 79 may form a composite field of view and/or a total field of view.

A field of view 79 may encompass the substrate 42, fully or at least part of it. The field of view 79 may be along the flow direction, e.g., in x-direction of Fig. 11. It may be provided that a first optical device 78 and/or camera is arranged upstream of the substrate 42 and/or such that its field of view 79 may cover a region upstream of the substrate 42. A second optical device 78 and/or camera may be arranged downstream of the substrate 42 and/or such that its field of view 79 may cover a region downstream of the substrate 42. The optical device 78 and/or camera may detect one or more of color, size, luminescence and granularity of the capture elements 30. Thereby, in some embodiments, multiplexing is enabled.

In some embodiments, determining a concentration of the analyte 10 is carried out by detection of jolting of capture elements 30, e.g., of magnetic capture elements 30, which may occur when bonding to at least one of the surface binder elements 44, cf. e.g. in the embodiment as shown in Figs. 11 to 13. The detection of jolting may be carried out by optical means, such as e.g. by employing the optical device 78 in Fig. 11.

When an analyte bonded capture element 30 is stuck and/or immobilized on the substrate 42, the binding interaction may impart a force onto the capture element 30 rolling along/on the substrate 42, such that the capture element 30 jolts. This jolting may be detected by the sensor unit 70, e.g., optically and/or by the optical device 78, cf., e.g., Fig. 11. In Fig. 12, the signal spikes correspond to a binding of an analyte bonded capture element 30 to at least one surface binder element 44, and/or to a jolt of a capture element 30.

Detecting the jolting of capture elements 30 may correspond to, may relate to and/or may correlate with differential counting. Thus, the concentration of the analyte 10 may be determined by detecting the jolting, and/or by counting the number of jolts. The analyte concentration may depend on, may be correlated with and/or may be a function of the jolting, and/or the detection of jolting may be employed to determine an analyte concentration.

In Fig. 13 the method according to the invention is compared to conventional assays. Whereas conventional assays may detect and/or may determine analyte concentrations over 2 to 3 orders of magnitude, the method and device according to the invention may detect and/or may determine analyte concentrations over 3 to 4 orders of magnitude, or even more. Thus, the method and the device according to the invention allow for a significantly improved detection and/or determination of the analyte concentration over a wide range, and provide assays with higher range of detection. It may be provided that the ratio and/or number of stuck and/or immobilized capture elements 30 of Fig. 22b, Fig. 23a and/or Fig. 23b is determined by detecting jolting of capture elements 30.

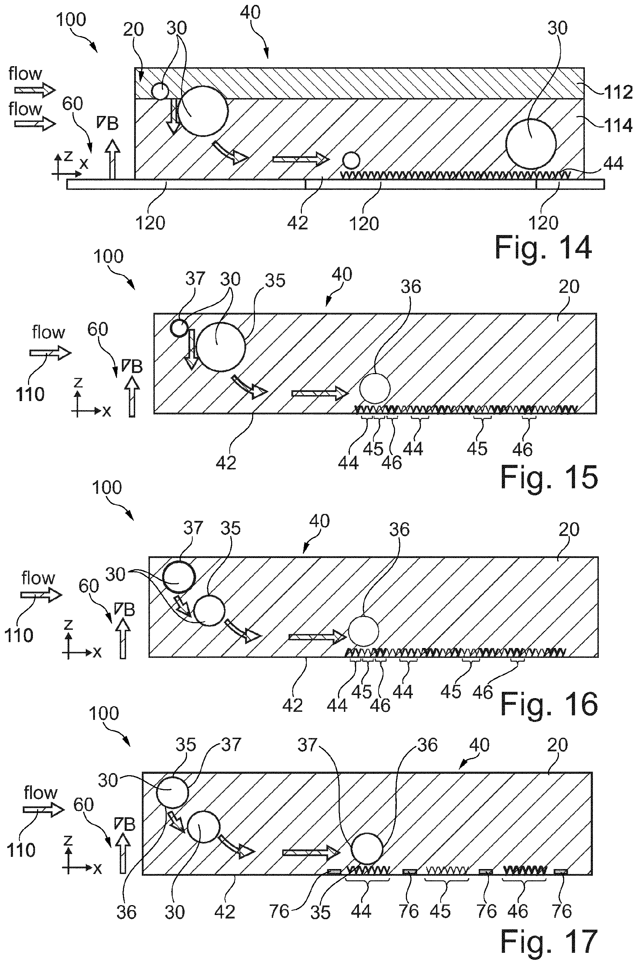

Figure 14 shows a device 100 according to an embodiment of the invention, where a laminated flow 114 is employed. The laminated flow may be or may comprise a sheath flow. The device 100 comprises a channel 40 with a substrate 42, e.g., a channel 40 and a substrate 42 as described above. The substrate 42 comprises surface binder elements 44. The device 100 may comprise an external force generating device 120, e.g., a magnetic field generating device 120. The external force generating device 120 may be configured to generate an external force 60 and/or a magnetic field.

The laminated flow114 may separate the sample flow 112 (which may correspond to the flow 110 of Figs. 1 to 13) from the substrate 42 in the channel 40. In some embodiments, the laminated flow 114 separates the sample flow 112 from the substrate 42 in the channel 40 along the full length of the channel 40, or at least partially. In some embodiments, the laminated flow 114 separates the sample flow 112 from the substrate 42 in the channel 40 at least over/above the substrate 42. In some embodiments, more than one laminated flow 114 is provided (not shown in Fig. 14). If more than one laminated flow 114 is provided, the laminated flows may be arranged and/or may flow over/above each other. The laminated flows may substantially flow in respective planes parallel to the surface of the substrate 42, where the planes may be arranged above each other with respect to a direction perpendicular to the surface of the substrate 42.

The external force 60, e.g., a magnetic force generated by a magnetic field, may be configured such that the capture elements 30, e.g., magnetic capture elements, are moved from the sample flow 112 into and/or through the laminated flow 114 towards the substrate 42. The external force 60 may pull or may attract the capture elements 30 from the sample flow 112 into and/or through the laminated flow 114 towards the substrate 42. A height of the laminated flow 114, e.g., in z-direction in Fig. 14, may be larger than the diameter of the capture elements 30, or the diameter of the largest of the capture elements 30.

By using a laminated flow 114, cellular or debris background from or in the sample 20 may be prevented from interfering with the rolling of the capture elements 30. The laminated flow 114 may reduce or may minimize interference from hematocrit.

Figs. 15 to 17 show embodiments of devices 100 and methods according to the invention which are configured and/or used for multiplexing. The method and device according to the invention may be suitable for carrying out heterogeneous assays. It may be provided that the sample 20 comprises or contains more than one, and/or multiple different kinds or types of analyte 10, at least one or multiple thereof, or respective concentrations, are to be detected and/or analyzed. For instance, in Figs. 15, 16 and/or 17, different exemplary embodiments which may determine the concentration of different types and/or kinds of analyte 10 are shown.

The respective devices 100 of Figs. 15, 16 and 17 comprise a respective channel 40 with a substrate 42, and a respective sensor unit 70 (not shown in Figs. 15 to 17). The devices 100 may comprise a respective external force generating device 120 (not shown in Figs. 15 to 17), e.g., a magnetic field generating device to generate a magnetic field. The embodiments as shown in Figs. 15, 16 and/or 17 may have one, more or all features and/or advantages of at least one other embodiment according to the invention. The capture elements 30 may be or comprise magnetic capture elements.

The substrate 42 may have different types and/or kinds of surface binder elements 44, 45, 46 for different types and/or kinds of analyte 10 to be detected and/or analyzed, cf., e.g., Figs. 15, 16 and 17. For instance, the substrate 42 may comprise first surface binder elements 44, second surface binder elements 45 and/or third surface binder elements 46. The first surface binder elements 44 may be configured to bind to a first kind and/or type of analyte 10, the second surface binder elements 45 may be configured to bind to a second kind and/or type of analyte 10, and/or the third surface binder elements 46 may be configured to bind to a third kind and/or type of analyte 10. The different kinds and/or types of surface binder elements 44, 45, 46 may be, e.g., arranged one behind the other, e.g., alternating, and/or in a substantially random arrangement. In some embodiments, the different kinds and/or types of surface binder elements 44, 45, 46 are arranged in homogeneous groups on portions of the substrate 42, cf., e.g., Fig. 17. It may be provided that the homogeneous groups are separated by a sensing element 76, cf., e.g., in the embodiment of Fig. 17.

The capture elements 30 may have different sizes or diameters, cf., e.g., Fig. 15. It may be provided that all capture elements 30 having substantially the same size or diameter have the same kind and/or type of capture binder elements 35, 36, 37 and/or may be configured to bind to the same type and/or kind of analyte 10. However, capture elements 30 of different size or diameter may have different kinds and/or types of capture binder elements 35, 36, 37 and/or may be configured to bind to different types and/or kinds of analyte 10. The capture elements 30 may be grouped into specific and/or distinct groups, according to their size or diameter. It may be provided that all capture elements 30 of the same group have the same kind and/or type of capture binder elements 35, 36, 37. It may be provided that all capture elements 30 of the same group have substantially the same size or diameter, are substantially identical or have substantially the same features.

For instance, capture elements 30 having a first size or diameter may have first capture binder elements 35, capture elements 30 having a second size or diameter may have second capture binder elements 36, and/or capture elements 30 having a third size or diameter may have third capture binder elements 37. The first capture binder elements 35, the second capture binder elements 36, and/or the third capture binder elements 37 may be different. Thus, the capture elements 30 of first size or diameter may be configured to bind to a first type and/or kind of analyte 10, the capture elements 30 of second size or diameter may be configured to bind to a second type and/or kind of analyte 10, and the capture elements 30 of third size or diameter may be configured to bind to a third type and/or kind of analyte 10.

Alternatively or additionally, the capture elements 30 may have different magnetic moments, cf., e.g., Fig. 16. For instance, some of the capture elements 30 may be or may comprise a different material than some other of the capture elements 30, such that their respective magnetic moments may differ.

The capture elements 30 may be grouped into specific and/or distinct groups, according to their magnetic moment. It may be provided that all capture elements 30 of the same group have the same kind and/or type of capture binder elements 35, 36, 37. It may be provided that all capture elements 30 of the same group have substantially the same magnetic moment, are substantially identical or have substantially the same features.

It may be provided that all capture elements 30 having substantially the same magnetic moment have the same kind and/or type of capture binder elements 35, 36, 37 and/or are configured to bind to the same type and/or kind of analyte 10. However, capture elements 30 of different magnetic moment may have different kinds and/or types of capture binder elements 35, 36, 37, and/or may be configured to bind to different types and/or kinds of analyte 10. For instance, capture elements 30 having a first magnetic moment may have first capture binder elements 35, capture elements 30 having a second magnetic moment may have second capture binder elements 36, and/or capture elements 30 having a third magnetic moment may have third capture binder elements 37. The first capture binder elements 35, the second capture binder elements 36, and/or the third capture binder elements 37 may be different. Thus, the capture elements 30 of first magnetic moment may be configured to bind to a first type and/or kind of analyte 10, the capture elements 30 of second magnetic moment may be configured to bind to a second type and/or kind of analyte 10, and the capture elements 30 of third magnetic moment may be configured to bind to a third type and/or kind of analyte 10.

It may be provided that some of the capture elements 30, or groups of the capture elements 30, have the same size, but differ in magnetic moment. It may be provided that some of the capture elements 30, or groups of the capture elements 30, have the same magnetic moment, but differ in size. Alternatively, some of the capture elements 30, or groups of the capture elements 30, may differ both in size and in magnetic moment.

When the capture elements 30 of specific size and/or of specific magnetic moment have specific capture binder elements 35, 36, 37 such as to bind to a specific analyte 10, the velocity decrease of the capture elements 30 of different size and/or of different magnetic moment may be employed to determine the concentration of the different specific analytes 10. Thus, multiplexing and/or differentiation between different analytes 10 is possible.

Capture elements 30 of different size or diameter may be distinguished by the sensor unit 70 and/or by the sensing elements 72, 74, 76. Similarly, capture elements 30 of different magnetic moment may be distinguished by the sensor unit 70 and/or by the sensing elements 72, 74, 76. Alternatively and/or additionally, an optical device 78 may be used, e.g. to determine the size and/or the velocity of the capture elements 30, and thus the associated analyte concentration.

Thus, referring, e.g., to Figs. 15 and 16, the concentration of the respective different types and/or kinds of analyte 10 may be determined by determining a decrease of the velocity of the capture elements 30 along the channel 40, and/or by determining a depletion of the capture elements 30 as described above. For this, there may be provided at least two sensing elements 72, 74 (not shown in Figs. 14, 15 and 16), e.g., the first sensing element 72 being arranged upstream of the surface binder elements 44, 45, 46 and the second sensing element 74 being arranged downstream of the surface binder elements 44, 45, 46. Since the sensor unit 70 and/or the sensing elements 72, 74 may distinguish the different kinds of capture elements 30 (e.g. as defined by their different size and/or different magnetic moment) a decrease of the velocity of the capture elements 30 and/or a depletion of each capture elements 30 of different size and/or of different magnetic moment may be detected, e.g. for each group of capture elements 30 of different size and/or of different magnetic moment may be detected. Since capture elements 30 of different size and/or of different magnetic moment are associated with different types and/or kinds of analyte 10, the concentration of analytes 10 may be determined as outlined above. Alternatively or additionally, an optical device 78 may be used, e.g. to determine the velocity, the number and/or the size of the capture elements 30 and thus the associated analyte concentration.

Alternatively or additionally, referring to Fig. 17, it may be provided that the capture elements 30, all of the capture elements 30 or at least some of the capture elements 30, have multiple different types and/or kinds of capture binder elements 35, 36, 37, or groups of different kinds and/or types of capture binder elements 35, 36, 37. It may be provided that the capture elements 30, all of the capture elements 30 or at least some of the capture elements 30, do neither differ in size or diameter nor in magnetic moment, but have multiple different types and/or kinds of capture binder elements 35, 36, 37, and/or groups of different kinds and/or types of capture binder elements 35, 36, 37. For instance, a capture element 30 may have one or more capture binder elements 35 of first type and/or kind, one or more capture binder elements 36 of second type and/or kind, and/or one or more capture binder elements 37 of third type and/or kind. The first capture binder elements 35, the second capture binder elements 36, and/or the third capture binder elements 37 may be different. The first capture binder elements 35 may be configured to bind to a first type and/or kind of analyte 10, the second capture binder elements 36 may be configured to bind to a second type and/or kind of analyte 10, and the third capture binder elements 37 may be configured to bind to a third type and/or kind of analyte 10. Correspondingly, the capture elements 30 may capture analytes 10 of first type and/or kind, analytes 10 of second type and/or kind, and/or analytes 10 of third type and/or kind.

In the embodiment of Fig. 17, multiplexing and/or differentiating between different analytes 10 are/is carried out by providing homogeneous groups of surface binder elements 44, 45, 46. The surface binder elements 44, 45, 46 of one specific homogeneous group may be configured to bind to one specific analyte 10. Then, the decrease of the velocity of the capture elements 30 and/or of the analyte bonded capture elements 30, along/on the substrate 42 in the regions of the specific homogeneous groups may be employed to determine the concentration of the corresponding specific analyte 10. Thus, multiplexing and/or differentiation between different analytes 10 are/is possible.

The decrease of the velocity of the capture elements 30 along the channel, and/or the depletion of the capture elements 30 may be determined by two sensing elements 76 separated by a homogeneous group of surface binder elements 44, 45, 46, respectively, as, e.g., seen in Fig. 17.

For instance, when a first type of analyte 10 may bind to first surface binder elements 44, capture elements 30 bound to the first analyte 10 may be slowed down and/or may be immobilized on the substrate 42 in the region of the homogeneous group of first surface binder elements 44. Thus, for determining the analyte concentration of a first type and/or kind of analyte 10, the decrease of the velocity of the capture elements 30 along the channel 40, and/or the depletion of the capture elements 30 may be determined as outlined above by the first sensing element 76 arranged upstream of and adjacent to the homogeneous group of first surface binder elements 44 and by the second sensing element 76 arranged downstream of and adjacent to the homogeneous group of first surface binder elements 44. In other words, the change and/or decrease of the velocity of the capture elements 30 along the channel 40, and/or the depletion of the capture elements 30 may be determined by the sensing elements 76 sandwiching the homogeneous group of first surface binder elements 44.

Similarly, when a second type of analyte 10 may bind to second surface binder elements 45, capture elements 30 bound to the second analyte 10 may be slowed down and/or may be immobilized on the substrate 42 in the region of the homogeneous group of second surface binder elements 45. Thus, for determining the analyte concentration of a second type and/or kind of analyte 10, the decrease of the velocity of the capture elements 30 along the channel 40, and/or the depletion of the capture elements 30 may be determined as outlined above by the first sensing element 76 arranged upstream of and adjacent to the homogeneous group of second surface binder elements 45 and by the second sensing element 76 arranged downstream of and adjacent to the homogeneous group of second surface binder elements 45. In other words, the decrease of the velocity of the capture elements 30 along the channel 40, and/or the depletion of the capture elements 30 may be determined by the sensing elements 76 sandwiching the homogeneous group of second surface binder elements 45.

Similarly, when a third type of analyte 10 may bind to third surface binder elements 46, capture elements 30 bound to the third analyte 10 may be slowed down and/or may be immobilized on the substrate 42 in the region of the homogeneous group of third surface binder elements 46. Thus, for determining the analyte concentration of a third type and/or kind of analyte 10, the decrease of the velocity of the capture elements 30 along the channel 40, and/or the depletion of the capture elements 30 may be determined as outlined above by the first sensing element 76 arranged upstream of and adjacent to the homogeneous group of third surface binder elements 46 and by the second sensing element 76 arranged downstream of and adjacent to the homogeneous group of third surface binder elements 46. In other words, the decrease of the velocity of the capture elements 30 along the channel 40, and/or the depletion of the capture elements 30 may be determined by the sensing elements 76 sandwiching the homogeneous group of third surface binder elements 46.

Alternatively or additionally, one or more optical devices 78 may be used, e.g., to determine the size, the velocity and/or the number of the capture elements 30, e.g., in the regions of the respective homogeneous groups of surface binder elements 44, 45, 46, and thus the associated analyte concentration.

While with reference to Figs. 15, 16 and 17 three different types and/or kinds of analyte 10, of capture elements 30, of capture binder elements 35, 36, 37 and/or of surface binder elements 44, 45, 46 are described, the number of analytes 10, capture elements 30, capture binder elements 35, 36, 37 and/or surface binder elements 44, 45, 46 is not limited thereto. Similarly, the arrangement of surface binder elements 44, 45, 46 may be different and/or not limited thereto. It may be provided that different capture elements 30 as described and or characterized above are mixed and/or used.

In some embodiments, at least one, multiple or all capture binder elements 35, 36, 37 is or comprises a tetraspanin, e.g., CD81, CD63 and/or CD9. In some embodiments, at least one, multiple or all surface binder elements 44, 45, 46 is or comprises a tetraspanin, e.g., CD81, CD63 and/or CD9.

It may be provided that the capture binder elements 35, 36, 37 differ, but the surface binder elements 44, 45, 46 may be the same, i.e. may be of same kind and/or type. In some embodiments, the surface binder elements 44, 45, 46 may be ubiquitous for all analytes 10 of interest.

In some embodiments, it may be provided that there is only one type and/or kind of capture binder elements 35, 36, 37, e.g. some type or kind of antibody for all capture binder elements 35, 36, 37, and only one type and/or kind of surface binder elements 44, 45, 46, e.g. some type or kind of antibody for all surface binder elements 44, 45, 46. The type and/or kind of the capture binder elements 35, 36, 37 may be the same as that of the surface binder elements 44, 45, 46. Alternatively, the type and/or kind of the capture binder elements 35, 36, 37 may be different from that of the surface binder elements 44, 45, 46. In some embodiments, it may be provided that the capture binder elements 35, 36, 37 of at least one, multiple or all capture elements 30 are of the same type and/or kind as at least some, multiple or all of the surface binder elements 44, 45, 46.

It may be provided that the capture binder elements 35, 36, 37 of all capture elements 30 are the same, i.e. are the same kind and/or type, but the surface binder elements 44, 45, 46 differ. In some embodiments, the capture binder elements 35, 36, 37 of at least one, multiple or all capture elements 30 match at least one, multiple or all of the surface binder elements 44, 45, 46, such that the respective capture binder elements 35, 36, 37 and the surface binder elements 44, 45, 46 form matched antibody pairs.

It may be provided that the embodiments as described with reference to Figs. 15, 16 and 17 are combined. For instance, the surface binder elements 44, 45, 46 may be arranged in homogeneous groups, and the size and/or the magnetic moments of the capture elements 30 may differ.

When the capture elements 30 are spherical or substantially sphere-shaped, they may roll along/on the substrate 42 such that substantially the same part of their surface repeatedly contacts the substrate 42 when rolling. For instance, the capture elements 30 may roll substantially along a circumferential disc on their surface. Thus, the analyte 10 bound to the capture binder elements 35, 36, 37 arranged outside said circumferential disc may not come into contact with the substrate 42 and/or with the surface binder elements 44, 45, 46, at all, or at least not as often as the analyte 10 bound to the capture binder elements 35, 36, 37 arranged on/within said circumferential disc.

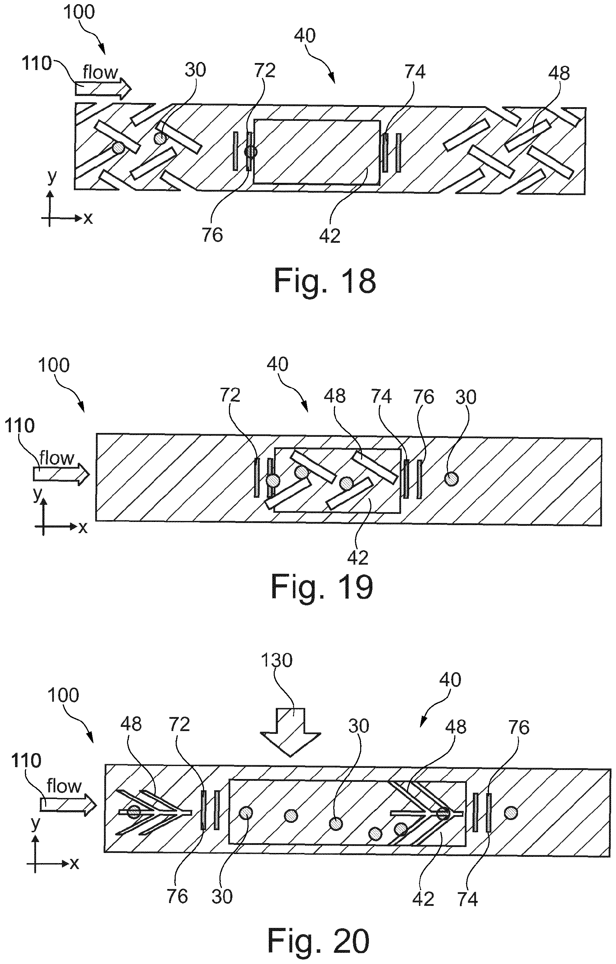

Thus, in some embodiments, it may be provided that the capture elements 30 rolling on the substrate 42 are manipulated, and/or that an orientation or a rolling direction of the capture elements is varied. Figs. 18, 19 and 20 show some corresponding exemplary embodiments according to the invention.

The embodiments as shown in Figs. 18, 19 and 20 comprise at least one, several and/or all features and/or advantages of a device 100 and/or of a method as described herein, for instance but not limited to those described above and/or shown in Figs. 1 to 17. Particularly, the embodiment as shown in Figs. 18, 19 and 20 comprise or include one or more of a channel 40, an external force generating device 120, e.g., a magnetic field generating device, an analyte 10 and/or a sample 20, e.g., as seen in Fig. 1 or 2, which are not shown in Figs. 18, 19 and/or 20. The capture elements 30 may be magnetic, and/or may comprise magnetic capture elements.

In some embodiments, the channel 40 has a pattern 48, as may be seen, e.g., in Figs. 18, 19 and 20. The pattern 48 may be configured to change, to manipulate or to vary the movement, the direction and/or the orientation of the capture elements 30, e.g., when flowing within the channel 40 and/or when rolling along/on the substrate 42. The pattern 48 may change, may manipulate or may vary the movement, the direction and/or the orientation of the capture elements 30 perpendicular or at least at an angle greater than 0 degrees, preferably 10 degrees or less, more preferably 6 degrees or less, relative to the flow direction, e.g. in x-direction in Figs. 18, 19, and 20. In some embodiments, said angle is maximally 15°, more preferably maximally 10°. The pattern 48 may deflect and/or may divert the capture elements 30 flowing within the channel 40 and/or rolling along/on the substrate 42. The pattern may exert a force onto a capture element 30 resulting in a change of movement, direction or orientation. The pattern 48 may be or may comprise a mechanical and/or a magnetic structure suitable for manipulating and/or varying the movement, the direction and/or the orientation of the capture elements 30 relative to the flow direction.

The pattern 48 may be a mechanical pattern 48. The mechanical pattern 48 may be and/or may comprise notches, recesses, ridges or the like. Alternatively or additionally, the pattern 48 may by a magnetic pattern 48. The magnetic pattern 48 may exert a magnetic force, e.g., as indicated by reference numeral 130 in Fig. 20, onto the capture elements 30 such as to manipulate or vary the movement, the direction or the orientation of the capture elements 30. The magnetic pattern 48 may modify or may modulate a magnetic field, e.g., a magnetic field provided by a magnetic field generating device.

In some embodiments, the pattern 48 is arranged in the channel 40, e.g., in and/or on an inner sidewall of the channel 40, but not overlapping with the substrate 42, cf. e.g. the exemplary embodiment as seen in Fig. 18. In some embodiments, the pattern 48 is arranged in the channel 40, e.g., in and/or on an inner sidewall of the channel 40, but not in and/or on the substrate 42, cf., e.g., Fig. 18. In some embodiments, the pattern 48 is arranged upstream and/or downstream of the substrate 42. In some embodiments, the pattern 48 only overlaps with the substrate 42, and/or is only on and/or in the substrate 42, cf., e.g., Fig. 19. Alternatively or additionally, the pattern 48 may at least partially overlap the substrate 42, and/or may be arranged on and/or in the substrate 42, cf., e.g., Figs. 19 or 20.

The pattern 48 may be or may comprise chevrons. The pattern 48 may be or may comprise a chevron pattern. The pattern 48 may be chevron-like or may have a chevron form.

Alternatively or additionally, the shape of a capture element 30 may deviate from a sphere, or may be not perfectly spherical (not shown in the figures). Then, the movement of such a capture element 30 when rolling may vary (during the time-of-roll), e.g., perpendicular to the flow direction such as in y-direction in Figs. 18, 19, and/or 20.

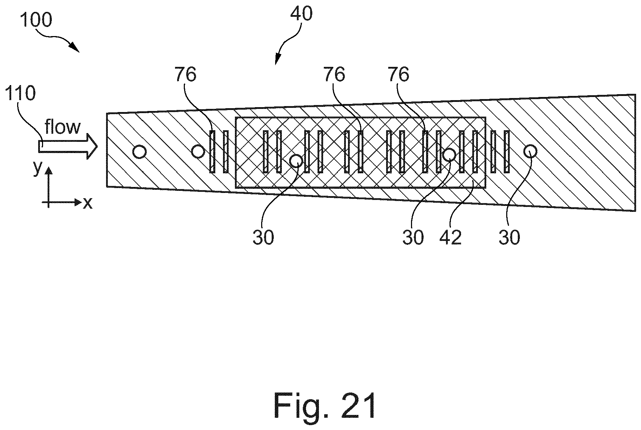

In some embodiments the channel 40 is tapered (cf. the exemplary embodiment as shown in Fig. 21), at least partially or completely. For instance, the channel 40 may be tapered in a direction perpendicular to the flow direction, e.g., perpendicular to the x-direction in Fig. 21. In some embodiments, the channel 40 is tapered in y-direction and/or in z-direction. When the channel 40 is tapered, the cross-section may vary. When the cross-section increases, the flow velocity may decrease. The channel 40 may be tapered such that its cross-section increases in flow direction, e.g., in x-direction in Fig. 21. In some embodiments, the capture elements 30 are guided and/or directed to the sensing unit 70 by the tapering of the channel 40. The channel 40 may be tapered such that the capture elements 30 may be guided and/or directed to the sensor unit 70, and/or to a sensing element 72, 74, 76.

When the channel 40 is tapered, the driving force on the capture elements 30 by the flow 110 may decrease, and the velocity of an analyte bonded capture element 30 may be further decreased. Alternatively or additionally, the likelihood of an analyte bonded capture element 30 to be immobilized and/or stuck may be increased.

In a preferred embodiment, the device 100 comprises a second external force generating device (not shown in the figures) which is configured to generate a second external force. The second external force generating device may be arranged downstream of the sensor unit 70, and/or downstream of the most downstream sensing element 74 or downstream of the most downstream optical device 78. In some embodiments, the second external force generating device is or comprises a second magnetic field generating device (not shown in the figures) and the second external force is a magnetic force. The second magnetic field generating device may be or may comprise a permanent magnet. The second external force generating device may be configured to generate the second external force varying in amplitude and/or spatial direction. The second external force may vary over time. It may be provided that the second external force generating device is a sorting unit for sorting capture elements 30. For instance, the second external force may be used to sort capture elements 30, e.g., sorting the capture elements 30 downstream of the sensor unit 70. By sorting, the capture elements 30 may be sorted into different groups. In some embodiments, different kinds/or types of analyte are bound to capture elements 30 of different groups. Capture elements 30 of different groups, and/or analyte bound to the capture elements 30 of the different groups, may be used for further analysis or diagnostics.

Figs 22 and 23 show exemplary measurements as carried out according to the disclosure. In Fig. 22a, the analyte 10 maybe or may comprise e.g. Her2neu+ EVs, the concentration of which in the sample has been assayed according to the disclosure. In Fig. 22a, the mean velocity difference as well as the mean delay of capture elements 30 has been measured with a method and/or a device according to the disclosure. In some embodiments, the capture binder elements 35, 36, 37 and/or the surface binder elements 42 are or comprise anti-Her2neu antibodies. The sample may be or may comprise a buffer, e.g., a suitable chemical. In Fig. 22a, the mean velocity difference corresponds to a change of velocity of the capture elements 30. The mean delay corresponds to a corresponding time of roll, as, e.g., determined by the sensor unit 70. As can be seen, the mean velocity difference and the mean delay increases monotonically with the concentration of the analyte 10. Thus, the concentration can be determined from the mean velocity difference and/or the mean delay.

In Fig. 22b, the analyte 10 may be or may comprises IL-6, the concentration of which in the sample has been assayed according to the disclosure. In Fig. 22b, the ratio of stuck and/or immobilized capture elements 30 (relative immobilized particles) is shown as function of the concentration of the analyte, here, e.g., IL-6, in the sample. The ratio of stuck and/or immobilized capture elements 30 can be determined, e.g., by differential counting, and/or by detection of jolting of the capture elements 30. The stuck and/or immobilized capture elements 30 have been determined according to the disclosure. In some embodiments, the capture binder elements 35, 36, 37 and/or the surface binder elements 42 are or comprise anti-IL6 antibodies. The sample may be or may comprise a buffer, such as e.g. a suitable chemical. As can be seen, the ratio of stuck and/or immobilized capture elements 30 increases monotonically with the concentration of the analyte 10. Thus, the concentration can be determined from the mean velocity difference and/or the mean delay. Moreover, the concentration can be determined in a range of nearly four decades and/or order of magnitude. For example, in Fig. 22b, the range of detection spans concentrations between around 1 ng/mL and 10000 ng/mL.

In Fig. 23a and fig. 23b, flow cytometry has been employed to determine the ratio of stuck and/or immobilized capture elements 30 (corresponding to the normalized signal). In Fig. 23a and 23b, optical flow cytometry and magnetic flow cytometry are compared. Fig. 23a shows the concentration of analyte 10 in a buffer, and Fig. 23b the concentration of the analyte 10 in a buffer (for the optical flow cytometry) and in a blood sample (for the magnetic flow cytometry). The ratio of stuck and/or immobilized capture elements 30 has been determined according to a method and/or device of the disclosure. The ratio of stuck and/or immobilized capture elements 30 can be determined, e.g., by differential counting, and/or by detection of jolting of the capture elements 30. In Fig. 23a and 23b, the ratio of stuck and/or immobilized capture elements 30 (relative immobilized particles) is shown as function of the concentration of the analyte, here biotin, in the sample. In some embodiments, the capture binder elements 35, 36, 37 and/or the surface binder elements 42 are or comprise avidin. As can be seen, the measured ratio of stuck and/or immobilized capture elements 30 as determined by the two respective measurements (i.e. by optical flow cytometry and magnetic flow cytometry) are in quite close agreement. In particular, the ratio of stuck and/or immobilized capture elements 30 can be determined accurately also for samples which are opaque (and where optical methods may fail), e.g., in blood. Thus, the magnetic flow cytometry is suitable, e.g., to determine the ratio (or number) of stuck and/or immobilized capture elements 30 in opaque samples, and thus also the concentration of analyte 10. As can be seen, the ratio of stuck and/or immobilized capture elements 30 increases monotonically with the concentration of the analyte 10. Thus, the concentration can be determined from the mean velocity difference and/or the mean delay. Moreover, the concentration can be determined in a range of nearly four decades and/or order of magnitude. For example, in Fig. 23a and 23b, the range of detection spans concentrations between around 0 mg/mL and 100 ng/mL.

The features as disclosed in the claims, the specification and the figures may be relevant for the realization of the invention in any combination thereof.

### List of reference numerals

- 10: analyte
- 20: sample
- 30: capture element
- 35: (first) capture binder element
- 36: second capture binder element
- 37: third capture binder element
- 40: channel
- 42: substrate
- 44: (first) surface binder element
- 45: second surface binder element
- 46: third surface binder element
- 48: pattern
- 60: external force
- 70: sensor unit
- 72: first sensing element
- 74: second sensing element
- 76: sensing element
- 78: optical device

- 100: device
- 110: flow
- 112: sample flow
- 114: laminated flow
- 120: external force generating device
- 130: force

## Claims

1. A method for detecting a concentration of at least one analyte (10) in a sample (20), the method comprising:
- mixing capture elements (30), preferably capture elements (30) comprising beads, more preferably comprising magnetic beads, into the sample (20), the capture elements (30) having capture binder elements (35, 36, 37);
- binding the analyte (10) to at least one capture element (30) via a capture binder element (35, 36, 37) such that at least one analyte bonded capture element (30) is formed;
- flowing the sample (20) through a channel (40) having a substrate (42), the substrate (42) having surface binder elements (44, 45, 46);
- moving the capture elements (30) to the substrate (42) with an external force (60), preferably with a magnetic force;
- rolling the capture elements (30) along/on the substrate (42) and changing the velocity of said at least one analyte bonded capture element (30) by an interaction, preferably by a binding interaction, between a surface binder element (44, 45, 46) of the substrate (42) and the analyte (10) bound by the respective capture binder element (35, 36, 37);
- determining a concentration of the analyte (10) using a sensor unit (70) by determining a change of velocity of the capture elements (30) along the channel (40) and/or by determining a depletion of the capture elements (30) along the channel (40), preferably by differential counting of the capture elements (30), and/or
determining binding kinetics, preferably binding constants, between the analyte (10) and the capture binder elements (35, 36, 37) and/or between the analyte (10) and the surface binder elements (44, 45, 46) wherein the binding kinetics are determined using the sensor unit (70) by determining a change of velocity of the capture elements (30) along the channel (40).

2. The method of claim 1, wherein determining the concentration of the analyte (10) comprises determining a time-of-roll between a first sensing element (72, 76) and a second sensing element (74, 76), wherein preferably the first sensing element (72, 76) is arranged upstream of the substrate (42), wherein preferably the second sensing element (74, 76) is arranged downstream of the substrate (42) and/or downstream of the first sensing element (72, 76), wherein preferably the first sensing element (72, 76) is or comprises a magnetic field sensor, and/or wherein preferably the second sensing element (74, 76) is or comprises a magnetic field sensor.

3. The method of any of the preceding claims, wherein determining the concentration of the analyte (10) comprises an optical detection, wherein the optical detection preferably comprises capturing a movement and/or a position of the capture elements (30) spatially and/or temporally resolved in a field of view (79).

4. The method of any of the preceding claims, wherein the external force (60) and a flow velocity of the sample (20) are adjusted such that at least one of the capture elements (30) rolls along/on the substrate (42), and/or wherein the external force (60) is adjusted such that the capture elements (30) are pushed away from the substrate (42), preferably after determining the concentration of the analyte (10).

5. The method of any of the preceding claims, wherein changing the velocity comprises immobilizing said at least one of the analyte bonded capture elements (30) by bonding the surface binder element (44, 45, 46) to the analyte (10) bound by the capture binder element (35, 36, 37).

6. The method of any of the preceding claims, wherein in the channel (40) a laminated flow (114) is flown between the sample (20, 112) and the substrate (42) such as to separate the sample (20, 112) from the substrate (42), and wherein the capture elements (30) are moved by the external force (60) from the sample (20, 112) through the laminated flow (114) to the substrate (42).

7. The method of any of the preceding claims, wherein at least one of the capture elements (30) has at least two different capture binder elements (35, 36, 37) each, wherein the different capture binder elements (35, 36, 37) are configured to bind to different analytes (10), and/or wherein the capture binder elements (35, 36, 37) of at least one or all capture elements (30) are of the same type and/or kind as at least one or all surface binder elements (44, 45, 46).

8. The method of any of the preceding claims, wherein at least two of the capture elements (30) have different sizes and different capture binder elements (35, 36, 37), and/or have different magnetic moments and different capture binder elements (35, 36, 37), and/or have different optical properties and different capture binder elements (35, 36, 37), wherein the different capture binder elements (35, 36, 37) are configured to bind to different analytes (10), and/or wherein the capture elements (30) comprise at least two groups of capture elements (30) and the capture elements (30) of each group have the same capture binder elements (35, 36, 37).

9. The method of any of the preceding claims, wherein the substrate (42) has at least two different surface binder elements (44, 45, 46), wherein the different surface binder elements (44, 45, 46) are configured to bind to different analytes (10), wherein preferably the different surface binder elements (44, 45, 46) are grouped into homogeneous groups, preferably separated by sensing elements (76).

10. The method of any of the preceding claims, wherein an orientation and/or a rolling direction of the capture elements (30) are/is varied when the capture elements (30) are rolling along/on the substrate (42).

11. A device (100) for detecting a concentration of at least one analyte (10) in a sample (20), the device (100) comprising:
a channel (40), wherein the channel (40) has at least one substrate (42), the substrate (42) having surface binder elements (44, 45, 46);
wherein the device (100) is configured such that, when the sample (20) that comprises the analyte (10) and capture elements (30) with capture binder elements (35, 36, 37), at least one of which capture elements (30) being bound to the analyte (10) via a capture binder element (35, 36, 37) forming an analyte bonded capture element (30), flows through the channel, an external force (60) moves the capture elements (30) to the substrate (42), such that the capture elements (30) roll along/on the substrate (42), wherein the surface binder elements (44, 45, 46) are configured to bind to the analyte (10) and/or to change the velocity of said at least one analyte bonded capture elements (30) by an interaction, preferably by a binding interaction, between at least one of the surface binder elements (44, 45, 46) and the analyte (10) bound by the respective capture binder element (35, 36, 37) when said at least one analyte bonded capture element (30) rolls along/on the substrate (42); and
a sensor unit (70) configured to determine a change of velocity of the capture elements (30) along the channel (40), and/or to determine a depletion of the capture elements (30) along the channel (40), preferably by differential counting of the capture elements (30), and/or to determine binding kinetics, preferably binding constants, between the analyte (10) and the capture binder elements (35, 36, 37) and/or between the analyte (10) and the surface binder elements (44, 45, 46).

12. The device (100) of claim 11, the device (100) comprising an external force generating device (120) configured to generate the external force (60), wherein preferably the external force generating device (120) is or comprises a magnetic field generating device and the external force (60) is a magnetic force, wherein preferably said magnetic field generating device is or comprises a permanent magnet.

13. The device (100) of claim 11 or 12, wherein the sensor unit (70) comprises a first sensing element (72, 76) and a second sensing element (74, 76), wherein the sensor unit (70) is configured to determine the concentration of the analyte (10) by determining a time-of-roll between the first sensing element (72, 76) and the second sensing element (74, 76), wherein preferably the first sensing element (72, 76) is arranged upstream of the substrate (42), wherein preferably the second sensing element (74, 76) is arranged downstream of the first sensing element (72, 76) and/or the substrate (42), wherein preferably the first sensing (72, 76) element and/or the second sensing element (74, 76) comprise/comprises or are/is a magnetic field sensor, respectively.

14. The device (100) of any of the preceding claims 11 to 13, wherein the sensor unit (70) is or comprises an optical device (78), wherein the optical device (78) is preferably configured to determine the concentration of the analyte (10) by capturing a movement and/or a position of the capture elements (30) spatially and/or temporally resolved in at least one field of view (79).

15. The device (100) of any of the preceding claims 11 to 14,
wherein the substrate (42) has at least two different surface binder elements (44, 45, 46), wherein the different surface binder elements (44, 45, 46) are configured to bind to different analytes (10), wherein preferably the different surface binder elements (44, 45, 46) are grouped into homogeneous groups, preferably separated by sensing elements (76), and/or
wherein the channel (40) and/or the substrate (42) have/has a mechanical and/or magnetic pattern (48), preferably a mechanical and/or magnetic chevron pattern, wherein the mechanical and/or magnetic pattern (48) are/is configured to vary an orientation and/or a rolling direction of the capture elements (30) when and/or before the capture elements (30) are rolling along/on the substrate (42), and/or
wherein the channel (40) is tapered, and/or
wherein the device (100) comprises a second external force generating device which is configured to generate a second external force and which is arranged downstream of the sensor unit (70), wherein preferably the second external force generating device is or comprises a second magnetic field generating device and the second external force is a magnetic force, wherein preferably said second magnetic field generating device is or comprises a permanent magnet, and/or wherein preferably the second external force generating device is configured to generate the second external force varying in amplitude and/or spatial direction.
